(19) Europäisches Patentamt
European Patent Office
Office européen des brevets

(11) Numéro de publication : **0 449 728 A1**

# (12) DEMANDE DE BREVET EUROPEEN

(21) Numéro de dépôt : **91400822.2**

(22) Date de dépôt : **27.03.91**

(51) Int. Cl.[5] : **C07D 495/14,** A61K 31/55, // (C07D495/14, 333:00, 243:00, 209:00)

(30) Priorité : **27.03.90 FR 9003839**

(43) Date de publication de la demande : **02.10.91 Bulletin 91/40**

(84) Etats contractants désignés : **AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Rault, Sylvain**
**Route de St Pierre sur Dives**
**F-14370 Moult (FR)**
Inventeur : **Boulouard, Michel**
**5 Boulevard du Général Vanier**
**F-14000 Caen (FR)**
Inventeur : **Robba, Max**
**4 rue Massillon**
**F-75004 Paris (FR)**
Inventeur : **Guardiola, Béatrice**
**6 rue Edouard Nortier**
**F-92200 Neuilly sur Seine (FR)**
Inventeur : **Devissaguet, Michelle**
**14 boulevard d'Inkermann**
**F-92200 Neuilly sur Seine (FR)**

## (54) Nouveaux dérivés de la 4H-pyrrolo (1,2-a)thieno(2,3-f) diazépine(1,4), leur procédé de préparation et les compositions pharmaceutiques les renfermant.

(57) Nouveaux composés de la 4H-pyrrolo-[1,2-a] thiéno[2,3-f] diazépine[1,4], utilisables comme médicament et répondant à la formule générale (I) :

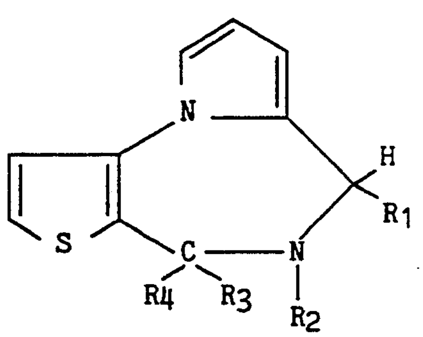

(I)

dans laquelle :

— $R_1$, $R_2$, $R_3$ et $R_4$ ont la même signification que dans les revendications.

Ces composés ainsi que leurs sels physiologiquement tolérables peuvent être utilisés en thérapeutique, notamment dans le traitement des troubles lies à l'hypoxémie et à certains désordres métaboliques.

EP 0 449 728 A1

La présente invention a pour objet de nouveaux composés de la 4H-pyrrolo[1,2-a] thiéno[2,3-f] diazépine [1,4], leur procédé de préparation et les compositions pharmaceutiques les renfermant.

Elle concerne particulièrement les composés de la 4H-pyrrolo [1,2-a] thiéno[2,3-f] diazépine[1,4] de formule générale (I) :

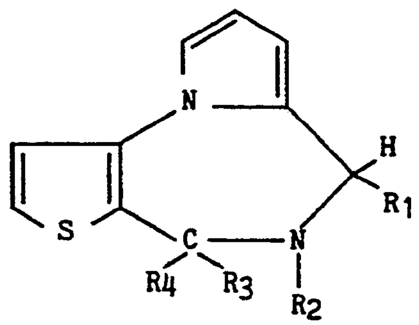

(I)

dans laquelle :

- $R_1$ représente :
  - un atome d'hydrogène,
  - un radical hydroxy,
  - un radical de formule générale :

$$-OR_5 \text{ ou } -SR_5$$

  dans laquelle $R_5$ représente :
  . un radical alkyle contenant de 1 à 5 atomes de carbone, en chaine droite ou ramifiée, éventuellement substitué par un radical phényle ou par un groupe de formule -COOB dans laquelle B représente un atome d'hydrogène ou un radical alkyle contenant de 1 à 5 atomes de carbone, ou
  . un radical aryle tel que par exemple un radical phényle,
  - un radical de formule générale :

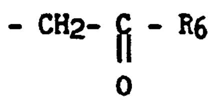

ou

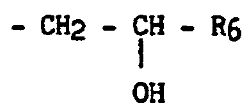

  dans laquelle $R_6$ représente :
  . un radical alkyle linéaire ou ramifié de 1 à 5 atomes de carbone,
  . un radical aryle (éventuellement substitué par un ou plusieurs atomes d'halogènes, groupements alcoxy de 1 à 3 atomes de carbone ou groupements trifluorométhyle ),
  . un radical aralkyle de 7 à 9 atomes de carbones (éventuellement substitué sur le noyau aromatique par un ou plusieurs atomes d'halogènes, groupements alcoxy de 1 à 3 atomes de carbone ou groupements trifluorométhyle),
  - un radical de formule :

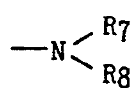

  dans laquelle :
  . $R_7$ et $R_8$, identiques ou différents, représentent chacun un atome d'hydrogène, un radical alkyle contenant de 1 à 5 atomes de carbone en chaine droite ou ramifiée, un radical cycloalkyle contenant de 3 à 5 atomes de carbone, un radical aryle (éventuellement substitué par un ou plusieurs atomes d'halogènes, groupements alcoxy de 1 à 3 atomes de carbone ou groupements trifluorométhyle) ou un radical aralkyle de 7 à 9 atomes de carbone (éventuellement substitué sur le noyau atomatique par un ou plusieurs atomes d'halogènes, groupements alcoxy de 1 à 3 atomes de carbone ou groupements trifluorométhyle) ou,
  . $R_7$ et $R_8$ forment ensemble avec l'atome d'azote auquel ils sont liés un radical hétérocyclique penta ou hexagonal renfermant éventuellement un autre hétéroatome tel par exemple un deuxième atome d'azote ou un atome d'oxygène, et éventuellement substitué par un radical alkyle linéaire ou ramifié de

1 à 5 atomes de carbone, par un radical alcoxycarbonyle de 2 à 6 atomes de carbone ou par un radical aryle, aralkyle de 7 à 9 atomes de carbone, hétéroaryle, diaralkyle comme par exemple le radical benzhydryle (éventuellement substitués sur le ou les noyaux aromatiques par un ou plusieurs substituants identiques ou différents choisis parmi halogène, hydroxy, alkyle ou alcoxy de 1 à 4 atomes de carbone, nitro ou trifluorométhyle),

– un radical alkyle renfermant de 1 à 5 atomes de carbone éventuellement substitué par un ou plusieurs radicaux choisis parmi le groupe formé des radicaux hydroxy, oxo, cyano, alkoxycarbonyle renfermant de 2 à 4 atomes de carbone ;

- $R_2$ représente :
  – un atome d'hydrogène,
  – un radical alkyle ayant de 1 à 5 atomes de carbone en chaine droite ou ramifiée,
  – un radical arylsulfonyle dans lequel le groupe aryle est éventuellement substitué par un radical alkyle ayant de 1 à 5 atomes de carbone en chaine droite ou ramifiée ;
  – un radical alkylcarbonyle de 2 à 3 atomes de carbone
  – un radical de formule générale :

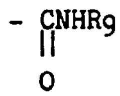

dans laquelle

. $R_9$ représente un radical alkyle linéaire ou ramifié, de 1 à 6 atomes de carbone, un radical aryle ou aralkyle de 7 à 9 atomes de carbone (éventuellement substitués sur le noyau aromatique par un ou plusieurs atomes d'halogène, radicaux alcoxy de 1 à 3 atomes de carbone ou radicaux trifluorométhyle),

- $R_3$ et $R_4$ représentent :
  – chacun simultanément un atome d'hydrogène ou,
  – ensemble un atome d'oxygène.

Selon la signification de $R_1$, les dérivés de formule générale (I) possèdent un ou plusieurs atomes de carbone asymétrique et de ce fait donnent des isomères ou des diastéréoisomères qui sont également inclus dans la présente invention.

L'état antérieur de la technique dans ce domaine est illustré notamment par : Heterocycles vol. 12 n° 8, pp. 1009-1011, (1979), Tetrahedron letters n° 7, pp. 643-644, (1979), et J. Heter. Chem., 14 n°2, pp. 235-240, (1977) qui mentionnent des dérivés de la pyrrolo[1,2-a] thiéno[3,2-f] diazépine[1,4] n'incluant ou ne suggérant nullement les composés de la présente invention.

Ces derniers qui sont des dérivés de la pyrrolo[1,2-a] thiéno [2,3-f] diazépine [1,4] de structure originale, possèdent des propriétés pharmacologiques intéressantes. Ils interfèrent notamment sur les récepteurs centraux des benzodiazépines de type périphérique et exercent un effet antihypoxique marqué, ce qui permet de les utiliser dans le traitement de syndromes ischémiques de toute localisation aigüe, transitoire ou progressive, et dans la correction des troubles liés à l'hypoxémie par exemple lors du vieillissement cérébral.

Ils possèdent également d'importantes propriétés hypocholestérolémiantes , hypotriglycéridémiantes, hypolipidémiantes et hypoglycémiantes qui les rendent précieux dans le traitement d'un grand nombre de troubles pathologiques.

La présente invention a également pour objet le procédé de préparation des composés de formule générale **(I)** caractérisé en ce que :

**A** - soit l'on traite l'aminocarbonyl-2 (formyl-2 pyrrolyl-1)-3 thiophène de formule **(II)** :

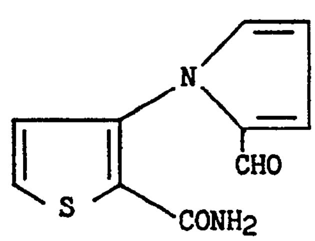

**(II)**

1) soit avec un mélange eau et triéthylamine, pour donner le composé de formule **(Ia)** :

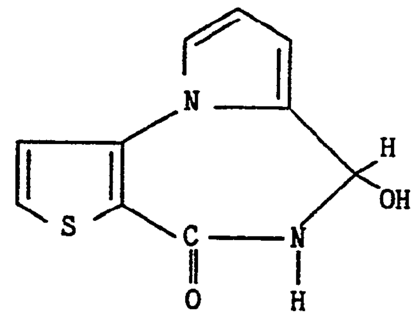

(Ia)

2) soit avec un alcool ou un thiol de formules générales respectives **(III)** et **(IV)** :

$R_5OH$ **(III)**, $R_5SH$ **(IV)**

dans lesquelles $R_5$ a la même signification que dans les composés de formule générale **(I)**, pour donner respectivement les composés de formules générales **(Ib)** et **(Ic)** :

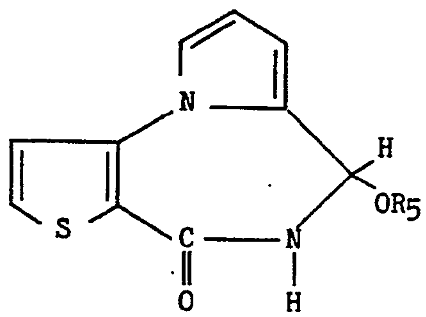

(Ib)

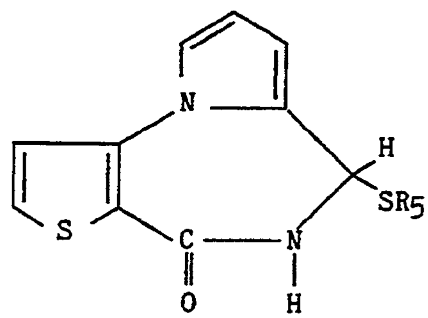

(Ic)

dans lesquelles $R_5$ a la signification précédemment définie ;

3) soit avec une amine secondaire de formule générale **(V)** :

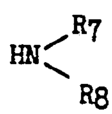

(V)

dans laquelle $R_7$ et $R_8$ ont les mêmes significations que dans les composés de formule générale **(I)**, pour donner les composés de formule générale **(Id)** :

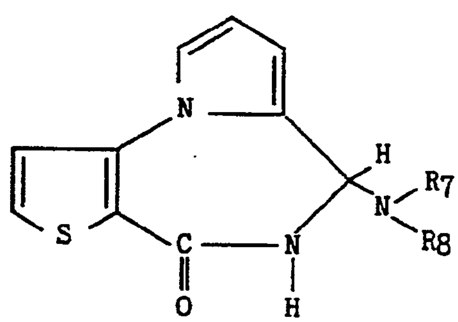

(Id)

dans laquelle $R_7$ et $R_8$ ont les mêmes significations que dans les composés de formule générale **(I)** ;

4) soit en milieu alacalin avec une méthylcétone de formule générale **(VI)** :

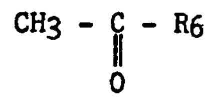

(VI)

dans laquelle $R_6$ a la même signification que dans les composés de formule générale **(I)** de manière à obtenir les composés de formule générale **(If)** :

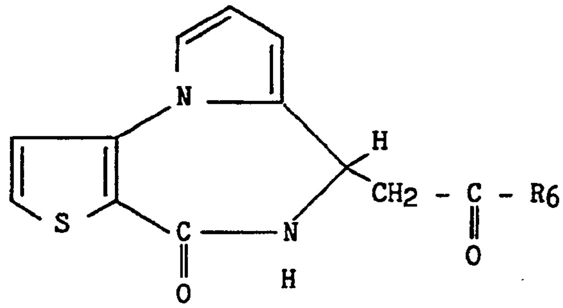

(If)

dans laquelle $R_6$ a la même signification que dans les composés de formule générale **(I)**, que l'on peut éventuellement traiter par un borohydrure de sodium pour obtenir les composés de formule générale **(Ig)** :

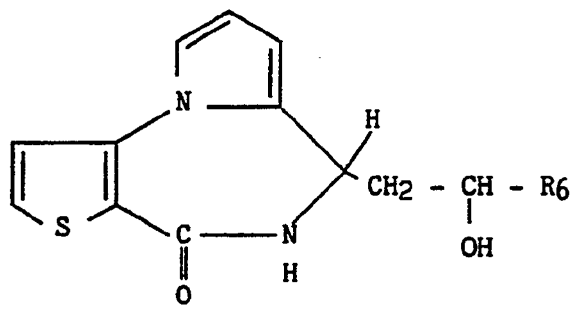

(Ig)

dans laquelle $R_6$ a la même signification que dans les composés de formule générale **(I)** ;

**B** - soit l'on traite le composé de formule générale **(Ia)** précédemment obtenu :

1) soit par un alcool de formule générale **(III)** précédemment définie pour donner les composés de formule générale **(Ib)** :

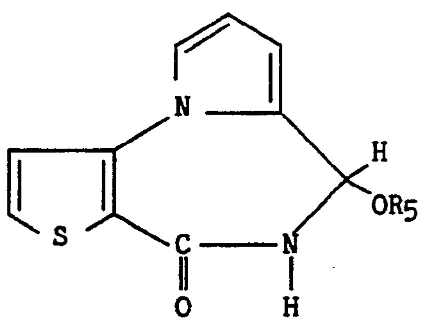

(Ib)

dans laquelle $R_5$ a la même définition que dans les composés de formule générale **(I)** ;

2) soit par une amine de formule générale **(V)** précédemment définie pour donner les composés de formule générale **(Id)** :

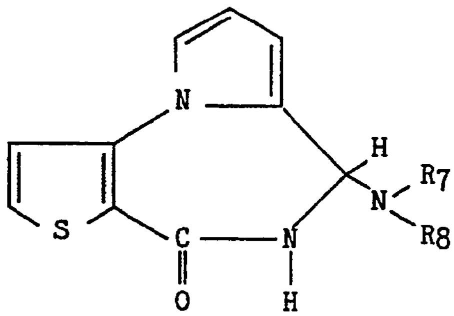

(Id)

dans laquelle $R_7$ et $R_8$ ont les mêmes significations que dans les composés de formule générale **(I)**, que l'on peut éventuellement traiter par le borohydrure de sodium pour obtenir le composé de formule **(Ie)** :

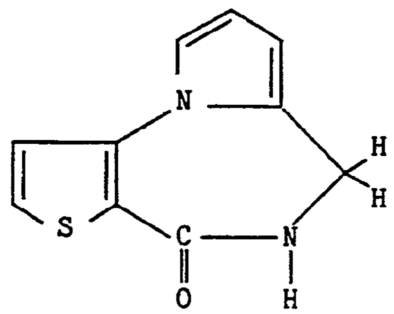

(Ie)

que l'on peut éventuellement faire réagir :

a) soit avec un anhydride de formule générale **(XI)** :

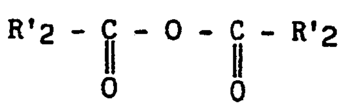

(XI)

dans laquelle $R'_2$ représente un radical méthyle ou éthyle de manière à obtenir les composés de formule générale **(Il)** :

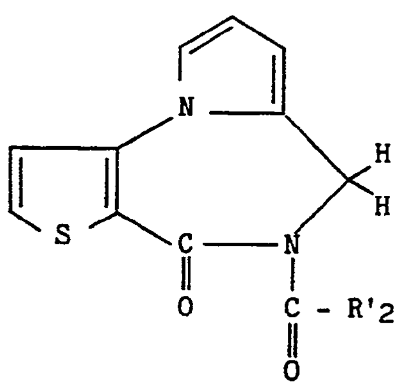

(Il)

dans laquelle $R'_2$ a la signification précedemment définie ;

b) soit avec un isocyanate de formule générale **(XII)** :

$$R_9 - N = C = O \qquad \textbf{(XII)}$$

dans laquelle $R_9$ a la même signification que dans les composés de formule générale **(I)** de manière à obtenir les composés de formule générale **(Im)** :

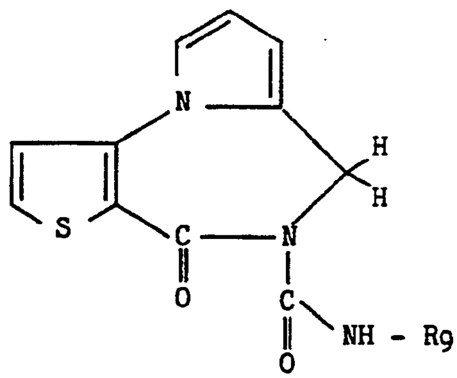

(Im)

dans laquelle $R_9$ à la même signification que dans les composés de formule générale **(I)**,

3) soit en milieu alcalin par une méthyl cétone, de formule générale **(VI)** précedemment définie pour donner les composés de formule générale **(If)** :

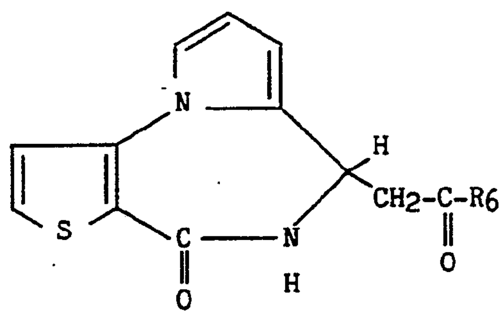

(If)

dans laquelle $R_6$ a la même signification que dans les composés de formule générale **(I)**,

4) soit, en présence de triéthylamine, par un composé de formule générale **(VII)** :

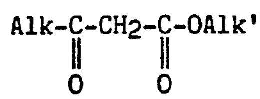

(VII)

dans laquelle Alk et Alk', identiques ou différents, représentent chacun un radical alkyle ayant de 1 à 3 atomes de carbone, pour donner les composés de formule générale **(Ih)** :

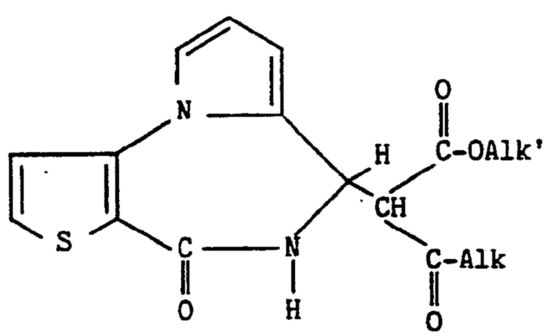

(Ih)

dans laquelle Alk et Alk' ont les significations précédemment définies ;

5) soit, en présence de triéthylamine, par un composé de formule générale **(VIII)** :

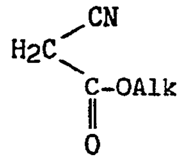

(VIII)

dans laquelle Alk a la signification précédemment définie, pour donner les composés de formule générale **(Ii)** :

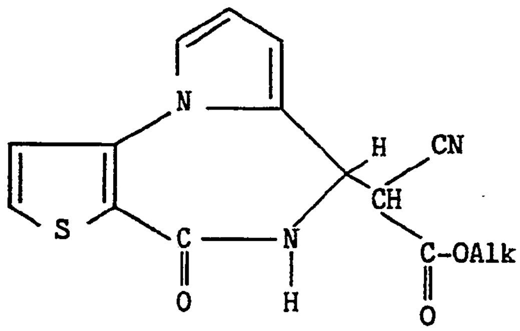

(Ii)

dans laquelle Alk a la signification précédemment définie ;
6) soit par $LiAlH_4$ pour donner le composé de formule **(Ij)** :

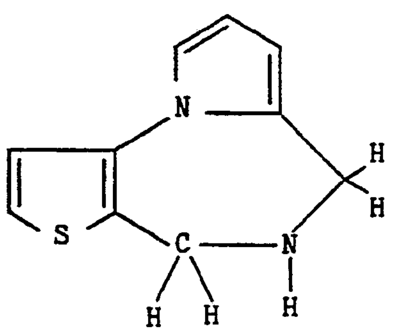

(Ij)

lequel traité

a) soit par une quantité stoechiométrique d'acide minéral ou organique, donne le sel d'addition acide correspondant ;

b) soit par un iodure d'alkyle de formule générale **(IX)** :

$R''_2$ I **(IX)**

dans laquelle $R''_2$ représente un radical alkyle ayant de 1 à 5 atomes de carbone en chaine droite ou ramifiée donne selon le rapport stoechiométrique **(IX)/(Ij)** :

soit le composé de formule générale **($Ik_1$)** :

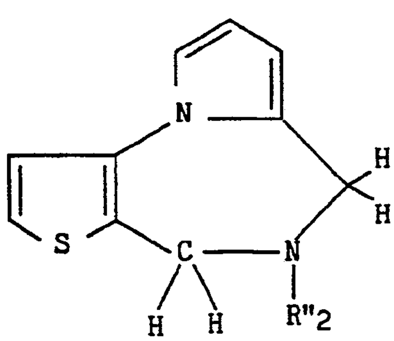

($Ik_1$)

dans laquelle $R''_2$ a la signification précédemment définie,

soit l'iodure de dialkylammonium de formule générale **(In)**

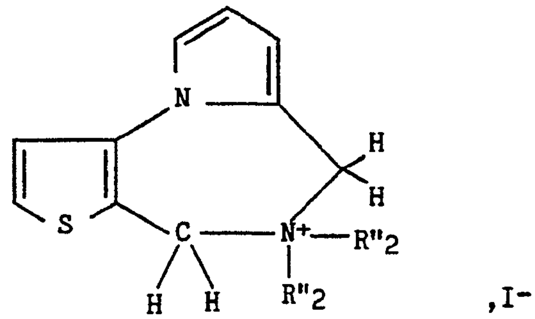

**(In)**

dans laquelle $R''_2$ a la signification précédemment définie,

c) soit par un composé halogéné de formule générale **(X)** :

Hal-R'' $'_2$ **(X)**

dans laquelle R'' '2 représente un radical arylsulfonyle dans lequel le groupe aryle est éventuellement substitué par un radical alkyle ayant de 1 à 5 atomes de carbone en chaine droite ou ramifiée, donne les composés de formule générale **($Ik_2$)**:

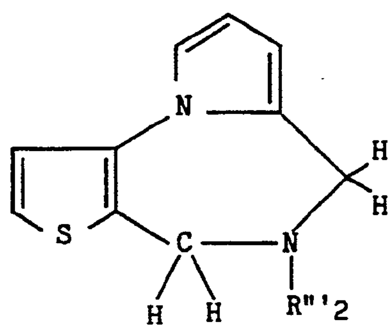

**(Ik2)**

dans laquelle R'' '2 a la signification précédemment définie.

L'ensemble des composés de formule **(Ia)** à **(Im)** font partie des composés de formule générale **(I)**.

Les composés de formule générale **(I)** donnent des sels avec les acides physiologiquement tolérables. Ces sels sont également inclus dans la présente invention.

Les composés de la présente invention ainsi que leurs sels possèdent des propriétés pharmacologiques et thérapeutiques intéressantes, notamment une puissante activité antihypoxique ainsi que d'importantes propriétés métaboliques.

Lors du vieillissement ou à la suite d'un accident vasculaire cérébral, la fragilité et la vulnérabilité cellulaire accrues sont des composantes physiopathologiques importantes pour la recherche de nouvelles thérapeutiques visant à protéger le cerveau mis en situation d'incapacité à répondre à toute nouvelle agression issue de son environnement.

Une telle agression peut être reproduite sous forme d'un défaut d'apport en oxygène et c'est pourquoi, par leurs conséquences, une analogie étroite existe entre l'hypoxie et le vieillissement cérébral.

Les composés de la présente invention ont été testés sur leur capacité à prolonger la survie du tissu cérébral lors d'hypoxie aiguë chez la souris. Ces essais ont prouvé que les composés de l'invention ont un effet protecteur antihypoxique très puissant et ont ainsi confirmé le grand intérêt de leur emploi en thérapeutique.

En s'opposant nettement à la mort cérébrale lors d'insuffisance d'apport en oxygène, les composés de la présente invention exercent un effet antihypoxique marqué et sont donc utiles dans les cas de syndromes ischémiques de toute localisation aiguë, transitoire ou progressive puisqu'ils exercent leurs propriétés pharmacologiques vis-à-vis de la défaillance d'oxygénation qui accompagne ces accidents. Leurs propriétés pharmacologiques permettent leur application dans la correction des désordres liés à l'hypoxémie par exemple lors du vieillissement cérébral.

Les composés de l'invention possèdent également de remarquables propriétés métaboliques en étant fortement hypolipidémiants, hypocholesterolémiants et hypotriglyceridémiants et hypoglycémiants.

Données pendant 15 jours à des rats soumis à un régime hypercholestérolémiant, ils se sont révélés être

beaucoup plus actifs (au moins 30 fois) que le Clofibrate sur l'abaissement des taux plasmatiques en triglycérides, cholestérol et HDL (high density lipoproteins)-cholestérol.

Par ailleurs les composés de la présente invention présentent chez l'animal, à la dose de 30 mg/Kg I.P. de bonnes propriétés hypotensives.

Les composés de formule générale (I) ainsi que leurs sels d'addition à un acide minéral ou organique pharmaceutiquement acceptable comme par exemple les acides chlorhydrique, méthanesulfonique, citrique, maléique peuvent être élaborés en préparations pharmaceutiques selon des procédés généralement connus comme par exemple en comprimés, gelules, dragées, solution buvable, solution injectable, suspensions buvables, émulsions, suppositoires.

Outre les excipients inertes, non toxiques et pharmaceutiquement acceptables tels par exemple l'eau distillée, le glucose, le lactose, l'amidon, le talc, les huiles végétales, l'éthylène glycol etc., ces préparations peuvent également contenir des agents de préservation, stabilisants, mouillants émulsifiants etc.

Les compositions ainsi obtenus se présentent généralement sous forme dosée et peuvent contenir selon les affections traitées, l'âge et le sexe du malade de 0,1 à 100 mg de principe actif. Elles peuvent selon le cas être administrées par voie orale, rectale ou parentérale à la dose de 0,1 à 100 mg de une à plusieurs fois par jour.

Les exemples suivants illustrent l'invention et ne la limitent en aucune façon.

Les paramètres de RMN relatifs aux dérivés exemplifiés sont regroupés dans les tableaux I - VIII.

**EXEMPLE 1 :**

**HYDROXY-6 DIHYDRO-5, 6 OXO-4 4H-PYRROLO[1,2-a] THIENO [2,3-f] DIAZEPINE[1,4 ] :**

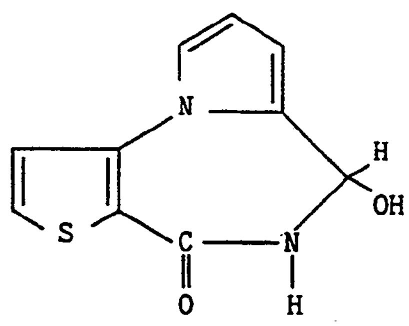

Une suspension de 1,5 g (0,0068 mole) d'aminocarbonyl-2 (formyl-2 pyrrolyl-1)-3 thiophène dans 30 ml d'eau et 0,75 ml de triéthylamine est agitée à température ambiante pendant 2 heures. Le précipité formé est essoré, lavé à l'eau et recristallisé dans l'éther éthylique.

On obtient 1 g d'hydroxy-6 dihydro-5,6 oxo-4 4H-pyrrolo[1,2-a] thiéno[2,3-f] diazépine[1,4] sous forme de cristaux jaunes fondant à 172°C.

Rendement : 67 %

Analyse élémentaire :

| | C % | H % | N % | S % |
|---|---|---|---|---|
| Calculé | 54,53 | 3,66 | 12,72 | 14,56 |
| Trouvé | 53,78 | 3,48 | 13,09 | 14,85 |

Spectre IR (KBr) :
- bande OH à 3520 $cm^{-1}$
- bandes NH à 3420 et 3280 $cm^{-1}$
- bande CO à 1640 $cm^{-1}$
- bandes principales à 1550, 1500, 1450, 1320, 1200, 1145, 870, 765 et 720 $cm^{-1}$.

**EXEMPLE 2 :**

**METHOXY-6 DIHYDRO-5, 6 OXO-4 4H-PYRROLO[1,2-a] THIENO[2,3-f] DIAZEPINE[1,4] :**

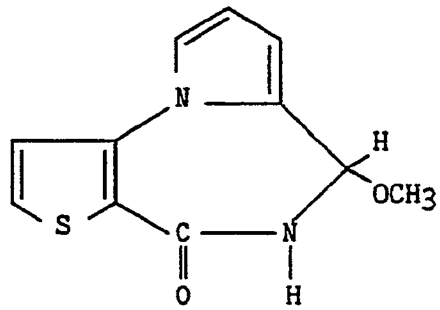

**Première méthode :**

Une suspension de 1 g (0,0045 mole) d'aminocarbonyl-2 (formyl-2 pyrrolyl-1)-3 thiophène dans 30 ml de méthanol est chauffé à reflux pendant une heure. Le méthanol est ensuite éliminé sous vide et le résidu solide recristallisé dans le méthanol.

On obtient 0,97 g de méthoxy-6 dihydro-5,6 oxo-4 4H-pyrrolo [1,2-a] thiéno[2,3-f] diazépine[1,4] sous forme de cristaux blancs fondant à 172°C.

Rendement : 92 %

Analyse élémentaire :

| | C % | H % | N % | S % |
|---|---|---|---|---|
| Calculé | 56,39 | 4,30 | 11,95 | 13,68 |
| Trouvé | 56,62 | 3,99 | 11,81 | 13,32 |

Spectre IR (KBr) :
- bande NH à 3250 $cm^{-1}$
- bandes CH à 3100, 2930 et 2830 $cm^{-1}$
- bande CO à 1650 $cm^{-1}$
- bandes principales à 1550, 1440, 1315, 1150, 1045, 730 et 715 $cm^{-1}$.

**Deuxième méthode :**

En opérant comme décrit dans la première méthode à partir de 1 g (0,0045 mole) d'hydroxy-6 dihydro-5,6 oxo-4 4H-pyrrolo[1,2-a] thiéno[2,3-f] diazépine[1,4] dans 30 ml de méthanol, on obtient 1 g de méthoxy-6 dihydro-5,6 oxo-4 4H-pyrrolo[1,2-a] thiéno[2,3-f] diazépine[1,4].

Rendement : 95 %

Le spectre infrarouge du composé ainsi obtenu est superposable à celui du composé préparé par la première méthode.

**EXEMPLES 3 - 6 :**

En opérant de façon analogue à celle décrite dans l'exemple 2, ont été préparés les dérivés suivants : (Dans tous les cas, quelle que soit la méthode de préparation utilisée, les spectres infrarouge des composés obtenus sont superposables.)

**3) ETHOXY-6 DIHYDRO-5,6 OXO-4 4H-PYRROLO[1,2-a] THIENO[2,3-f] DIAZEPINE[1,4],** sous forme de cristaux blancs fondant à 148°C (éthanol),

Analyse élémentaire :

| | C % | H % | N % | S % |
|---|---|---|---|---|
| Calculé | 58.05 | 4,87 | 11,28 | 12,91 |
| Trouvé | 57,83 | 4,80 | 11,20 | 12,79 |

Spectre IR (KBr) :
- bandes NH à 3260 et 3180 $cm^{-1}$
- bandes CH à 3060, 2970, 2930 et 2870 $cm^{-1}$
- bande CO à 1635 $cm^{-1}$
- bandes principales à 1550, 1500, 1440, 1325, 1235, 1045, 960, 735 et 715 $cm^{-1}$,

à partir :

a) d'aminocarbonyl-2 (formyl-2 pyrrolyl-1)-3 thiophène et d'éthanol, avec un rendement de 86 %, et

b) d'hydroxy-6 dihydro-5,6 oxo-4 4H-pyrrolo[1,2-a] thiéno[2,3-f] diazépine[1,4] et d'éthanol, avec un rendement de 89 %.

**4) PROPOXY-6 DIHYDRO-5,6 OXO-4 4H-PYRROLO[1,2-a] THIENO[2,3-f] DIAZEPINE[1,4],** sous forme d'aiguilles blanches, fondant à 164°C (éther éthylique),

Analyse élémentaire :

| | C % | H % | N % | S % |
|---|---|---|---|---|
| Calculé | 59,52 | 5,38 | 10,68 | 12,22 |
| Trouvé | 59,59 | 5,33 | 10,72 | 12,08 |

Spectre IR (KBr) :
- bande NH à 3300 $cm^{-1}$
- bandes CH à 3120, 2960, 2940 et 2880 $cm^{-1}$
- bande CO à 1645 $cm^{-1}$
- bande CN à 1605 $cm^{-1}$
- bandes principales à 1500, 1450, 1325, 1150, 1060, 740 et 720 $cm^{-1}$,

à partir :

a) d'aminocarbonyl-2 (formyl-2 pyrrolyl-1)-3 thiophène et de propanol, avec un rendement de 75 %, et

b) d'hydroxy-6 dihydro-5,6 oxo-4 4H-pyrrolo[1,2-a] thiéno[2,3-f] diazépine[1,4] et de propanol, avec un rendement de 80 %.

**5) (METHYL-1 ETHOXY)-6 DIHYDRO-5,6 OXO-4 4H-PYRROLO[1,2-a] THIENO[2,3-f] DIAZEPINE[1,4],** sous forme de cristaux blancs fondant à 160°C (isopropanol),

Analyse élémentaire :

| | C % | H % | N % | S % |
|---|---|---|---|---|
| Calculé | 59,52 | 5,37 | 10,67 | 12,22 |
| Trouvé | 59,44 | 5,30 | 10,62 | 12,06 |

Spectre IR (KBr) :
- bandes NH à 3260 et 3170 $cm^{-1}$
- bandes CH à 3100, 3050, 2960 et 2880 $cm^{-1}$
- bande CO à 1640 $cm^{-1}$

– bandes principales à 1550, 1500, 1460, 1440, 1325, 1110, 1020, 805 et 720 $cm^{-1}$,

à partir :

a) d'aminocarbonyl-2 (formyl-2 pyrrolyl-1)-3 thiophène et d'isopropanol, avec un rendement de 80 %, et

b) d'hydroxy-6 dihydro-5,6 oxo-4 4H-pyrrolo[1,2-a] thiéno[2,3-f] diazépine[1,4] et d'isopropanol, avec un rendement de 76 %.

**6) BENZYLOXY-6 DIHYDRO-5,6 OXO-4 4H-PYRROLO[1,2-a] THIENO[2,3-f] DIAZEPINE[1,4],** sous forme de cristaux blancs fondant à 179°C (éther éthylique),

<u>Analyse élémentaire</u> :

| | C % | H % | N % | S % |
|---|---|---|---|---|
| Calculé | 65,79 | 4,55 | 9,02 | 10,33 |
| Trouvé | 65,31 | 4,71 | 9,22 | 10,66 |

<u>Spectre IR (KBr)</u> :

– bandes NH à 3270 et 3190 $cm^{-1}$

– bandes CH à 3060, 2940 et 2880 $cm^{-1}$

– bande CO à 1645 $cm^{-1}$

– bandes principales à 1540, 1495, 1450, 1320, 1205, 1015, 795 et 735 $cm^{-1}$,

à partir :

a) d'aminocarbonyl-2 (formyl-2 pyrrolyl-1)-3 thiophène et d'alcool benzylique, avec un rendement de 60 %, et

b) d'hydroxy-6 dihydro-5,6 oxo-4 4H-pyrrolo[1,2-a] thiéno[2,3-f] diazépine[1,4] et d'alcool benzylique, avec un rendement de 64 %.

<u>**EXEMPLE 7 :**</u>

**METHOXYCARBONYLMETHYLMERCAPTO-6 DIHYDRO-5,6 OXO-4 4H-PYRROLO [1,2-a] THIENO[2,3-f] DIAZEPINE[1,4] :**

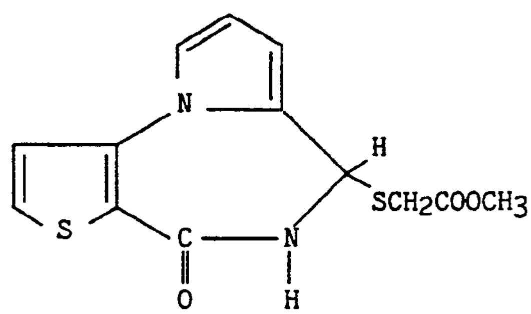

Une solution de 0,7 g (0,0032 mole) d'aminocarbonyl-2 (formyl-2 pyrrolyl-1)-3 thiophène dans 80 ml d'acétonitrile est additionnée de 0,3 ml (0,0035 mole) de thioglycolate de méthyle. Le mélange réactionnel est agité à température ambiante pendant 12 heures. L'acétonitrile est ensuite éliminé sous vide. L'huile résiduelle qui cristallise par addition d'éther de pétrole est recristallisée.

On obtient 0,65 g de méthoxycarbonylméthylmercapto-6 dihydro-5,6 oxo-4 4H-pyrrolo[1,2-a] thiéno[2,3-f] diazépine[1,4], sous forme de cristaux blancs fondant à 158°C (éther éthylique).

Rendement : 68 %

Analyse élémentaire :

| | C % | H % | N % | S % |
|---|---|---|---|---|
| Calculé | 50,64 | 3,92 | 9,08 | 20,79 |
| Trouvé | 50,55 | 3,67 | 8,97 | 20,71 |

Spectre IR (KBr) :
- bandes NH à 3270 et 3160 $cm^{-1}$
- bandes CH à 3050, 3010 et 2920 $cm^{-1}$
- bandes CO à 1715 $cm^{-1}$ (ester) et 1645 $cm^{-1}$ (lactame)
- bandes principales à 1545, 1490, 1450, 1440, 1310, 1220, 1145, 755 et 720 $cm^{-1}$.

**EXEMPLES 8 - 9 :**

En opérant de façon analogue à celle décrite dans l'exemple 7, ont été préparés les dérivés suivants :

**8) PHENYLMERCAPTO-6 DIHYDRO-5,6 OXO-4 4H-PYRROLO[1,2-a] THIENO[2,3-f] DIAZEPINE[1,4],** sous forme d'aiguilles blanches fondant à 178°C (éther éthylique),

Analyse élémentaire :

| | C % | H % | N % | S % |
|---|---|---|---|---|
| Calculé | 61,51 | 3,87 | 8,97 | 20,52 |
| Trouvé | 61,70 | 3,96 | 9,10 | 20,54 |

Spectre IR (KBr) :
- bandes NH à 3270 et 3160 $cm^{-1}$
- bandes CH à 3180 et 3010 $cm^{-1}$
- bande CO à 1645 $cm^{-1}$
- bandes principales à 1550, 1500, 1480, 1440, 1395, 1330, 1065, 775, 720 et 690 $cm^{-1}$,

à partir d'aminocarbonyl-2 (formyl-2 pyrrolyl-1)-3 thiophène et de thiophénol, avec un rendement de 72 %.

**9) CARBOXYETHYLMERCAPTO-6 DIHYDRO-5,6 OXO-4 4H-PYRROLO[1,2-a] THIENO[2,3-f] DIAZEPINE[1,4],** sous forme de cristaux blancs fondant à 190°C (éther éthylique),

Analyse élémentaire :

| | C % | H % | N % | S % |
|---|---|---|---|---|
| Calculé | 50,64 | 3,92 | 9,08 | 20,79 |
| Trouvé | 50,53 | 3,89 | 9,09 | 20,79 |

Spectre IR (KBr) :
- bande OH à 3450 $cm^{-1}$
- bande NH à 3240 $cm^{-1}$
- bandes CH à 3150, 3020 et 2930 $cm^{-1}$
- bandes CO à 1700 $cm^{-1}$ (acide) et 1610 $cm^{-1}$ (lactame)
- bandes principales à 1500, 1450, 1330, 1195, 770 et 720 $cm^{-1}$,

à partir d'aminocarbonyl-2 (formyl-2 pyrrolyl-1)-3 thiophène et d'acide mercapto-3 propionique, avec un rendement de 43 %.

**EXEMPLE 10 :**

**METHYLAMINO-6 DIHYDRO-5,6 OXO-4 4H-PYRROLO[1,2-a] THIENO[2,3-f] DIAZEPINE[1,4] :**

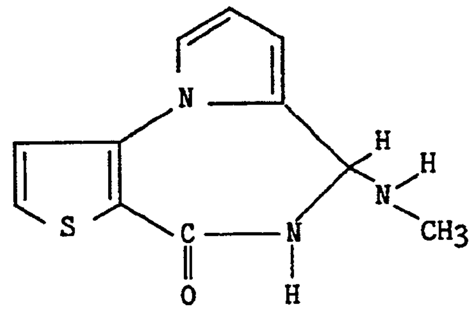

A une suspension de 1,1 g (0,005 mole) d'hydroxy-6 dihydro-5,6 oxo-4 4H-pyrrolo[1,2-a] thiéno[2,3-f] diazépine[1,4] dans 20 ml d'eau est ajouté en une seule fois 3 ml d'une solution aqueuse de méthylamine à 35 %, puis le mélange réactionnel est agité pendant 4 heures à température ambiante. Le produit se solubilise progressivement puis reprécipite. Le précipité est essoré, lavé à l'eau, séché et recristallisé.

On obtient 0,7 g de méthylamino-6 dihydro-5,6 oxo-4 4H-pyrrolo[1,2-a] thiéno[2,3-f] diazépine[1,4], sous forme de cristaux blancs fondant à 146°C (éther éthylique).

Rendement : 60 %

Analyse élémentaire :

| | C % | H % | N % | S % |
|---|---|---|---|---|
| Calculé | 56,63 | 4,75 | 18,01 | 13,74 |
| Trouvé | 56,28 | 4,51 | 17,91 | 13,18 |

Spectre IR (KBr) :
- bandes NH à 3320, 3250 et 3180 $cm^{-1}$
- bandes CH à 3040, 2980 et 2900 $cm^{-1}$
- bande CO à 1625 $cm^{-1}$
- bandes principales à 1550, 1495, 1440, 1320, 1100, 770 et 710 $cm^{-1}$.

**EXEMPLES 11 - 19 :**

En opérant de façon analogue à celle décrite dans l'exemple 10, ont été préparés les dérivés suivants :

**11) ETHYLAMINO-6 DIHYDRO-5,6 OXO-4 4H-PYRROLO[1,2-a] THIENO[2,3-f] DIAZEPINE[1,4]**, sous forme de cristaux blancs fondant à 152°C (éther éthylique),

Analyse élémentaire :

| | C % | H % | N % | S % |
|---|---|---|---|---|
| Calculé | 58,28 | 5,3 | 16,99 | 12,96 |
| Trouvé | 58,21 | 5,26 | 16,58 | 12,48 |

Spectre IR (KBr) :
- bandes NH à 3290 et 3150 $cm^{-1}$
- bandes CH à 3050, 2960 et 2860 $cm^{-1}$
- bande CO à 1630 $cm^{-1}$

– bandes principales à 1550, 1490, 1450, 1320, 1200, 760 et 720 $cm^{-1}$,
à partir d'hydroxy-6 dihydro-5,6 oxo-4 4H-pyrrolo[1,2-a] thiéno[2,3-f] diazépine[1,4] et d'éthylamine, avec un rendement de 72 %.

**12) PROPYLAMINO-6 DIHYDRO-5,6 OXO-4 4H-PYRROLO[1,2-a] THIENO[2,3-f] DIAZEPINE[1,4]**, sous forme de cristaux blancs fondant à 130°C (éther éthylique),

Analyse élémentaire :

| | C % | H % | N % | S % |
|---|---|---|---|---|
| Calculé | 59,75 | 5,78 | 16,08 | 12,27 |
| Trouvé | 59,63 | 5,82 | 15,95 | 12,13 |

Spectre IR (KBr) :
– bandes NH à 3340, 3260 et 3170 $cm^{-1}$
– bandes CH à 3100, 3010, 2960 et 2860 $cm^{-1}$
– bande CO à 1625 $cm^{-1}$
– bandes principales à 1540, 1495, 1435, 1330, 1095 et 740 $cm^{-1}$,
à partir d'hydroxy-6 dihydro-5,6 oxo-4 4H-pyrrolo[1,2-a] thiéno[2,3-f] diazépine[1,4] et de propylamine, avec un rendement de 85 %.

**13) PYRROLIDINO-6 DIHYDRO-5,6 OXO-4 4H-PYRROLO[1,2-a] THIENO[2,3-f] DIAZEPINE[1,4]**, sous forme de cristaux blancs fondant à 191°C (éther éthylique),

Analyse élémentaire :

| | C % | H % | N % | S % |
|---|---|---|---|---|
| Calculé | 61,52 | 5,53 | 15,37 | 11,73 |
| Trouvé | 61,62 | 5,41 | 15,35 | 11,79 |

Spectre IR (KBr) :
– bandes NH à 3260 et 3190 $cm^{-1}$
– bandes CH à 3050, 2960 et 2800 $cm^{-1}$
– bande CO à 1620 $cm^{-1}$
– bandes principales à 1550, 1495, 1390, 1305, 1125, 870 et 710 $cm^{-1}$,
à partir d'hydroxy-6 dihydro-5,6 oxo-4 4H-pyrrolo[1,2-a] thiéno[2,3-f] diazépine[1,4] et de pyrrolidine, avec un rendement de 93 %.

**14) PIPERIDINO-6 DIHYDRO-5,6 OXO-4 4H-PYRROLO[1,2-a] THIENO[2,3-f] DIAZEPINE[1,4]**, sous forme de cristaux blancs fondant à 178°C (éther éthylique),

Analyse élémentaire :

| | C % | H % | N % | S % |
|---|---|---|---|---|
| Calculé | 62,69 | 5,96 | 14,62 | 11,16 |
| Trouvé | 62,65 | 5,96 | 14,57 | 11,20 |

Spectre IR (KBr) :
– bandes NH à 3260 et 3170 $cm^{-1}$
– bandes CH à 3050, 2940, 2860 et 2810 $cm^{-1}$
– bande CO à 1640 $cm^{-1}$
– bandes principales à 1550, 1500, 1460, 1310, 1230, 1150, 980, 770, 740 et 720 $cm^{-1}$,

à partir d'hydroxy-6 dihydro-5,6 oxo-4 4H-pyrrolo[1,2-a] thiéno[2,3-f] diazépine[1,4] et de pipéridine, avec un rendement de 89 %.

**15) MORPHOLINO-6 DIHYDRO-5,6 OXO-4 4H-PYRROLO[1,2-a] THIENO[2,3-f] DIAZEPINE[1,4],** sous forme de cristaux transparents fondant à 198°C (éther éthylique),

Analyse élémentaire :

| | C % | H % | N % | S % |
|---|---|---|---|---|
| Calculé | 58,11 | 5,22 | 14,52 | 11,08 |
| Trouvé | 57,92 | 5,23 | 14,46 | 10,95 |

Spectre IR (KBr) :
- bandes NH à 3270 et 3170 $cm^{-1}$
- bandes CH à 3050, 2950, 2930 et 2840 $cm^{-1}$
- bande CO à 1640 $cm^{-1}$
- bandes principales à 1500, 1460, 1310, 1120, 990, 875 et 715 $cm^{-1}$,

à partir d'hydroxy-6 dihydro-5,6 oxo-4 4H-pyrrolo[1,2-a] thiéno[2,3-f] diazépine[1,4] et de morpholine, avec un rendement de 92 %.

**16) CYCLOPROPYLAMINO-6 DIHYDRO-5,6 OXO-4 4H-PYRROLO[1,2-a] THIENO[2,3-f] DIAZEPINE[1,4],** sous forme de cristaux blancs fondant à 185°C (éther éthylique),

Analyse élémentaire :

| | C % | H % | N % | S % |
|---|---|---|---|---|
| Calculé | 60,21 | 5,05 | 16,20 | 12,36 |
| Trouvé | 60,67 | 5,30 | 15,87 | 11,90 |

Spectre IR (KBr) :
- bandes NH à 3340, 3260 et 3150 $cm^{-1}$
- bandes CH à 3020, 2960 et 2880 $cm^{-1}$
- bande CO à 1630 $cm^{-1}$
- bandes principales à 1540, 1490, 1460, 1370, 1290,1120, 870, 790, 735 et 720 $cm^{-1}$,

à partir d'hydroxy-6 dihydro-5,6 oxo-4 4H-pyrrolo[1,2-a] thiéno[2,3-f] diazépine[1,4] et de cyclopropylamine, avec un rendement de 86 %.

**17) (ETHOXYCARBONYL 4-PIPERAZINO)-6 DIHYDRO-5,6 OXO-4 4H-PYRROLO[1,2-a] THIENO[2,3-f] DIAZEPINE[1,4],** sous forme de cristaux blancs fondant à 180°C (éther éthylique),

Analyse élémentaire :

| | C % | H % | N % | S % |
|---|---|---|---|---|
| Calculé | 56,65 | 5,59 | 15,54 | 8,89 |
| Trouvé | 56,76 | 5,48 | 15,47 | 8,78 |

Spectre IR (KBr) :
- bandes NH à 2260 et 3160 $cm^{-1}$
- bandes CH à 3110, 2980 et 2810 $cm^{-1}$
- bandes CO à 1690 $cm^{-1}$ (ester) et 1640 $cm^{-1}$ (lactame)
- bandes principales à 1515, 1495, 1250, 1225, 985, 870, 770 et 710 $cm^{-1}$,

à partir d'hydroxy-6 dihydro-5,6 oxo-4 4H-pyrrolo[1,2-a] thiéno[2,3-f] diazépine[1,4] et d'éthoxycarbonyl-4 pipérazine, avec un rendement de 80 %.

**18) (PHENYL-4 PIPERAZINO)-6 DIHYDRO-5,6 OXO-4 4H-PYRROLO[1,2-a] THIENO[2,3-f] DIAZEPINE[1,4]**, sous forme de cristaux blancs fondant à 150°C (éther éthylique),

Analyse élémentaire :

| | C % | H % | N % | S % |
|---|---|---|---|---|
| Calculé | 65,91 | 5,53 | 15,37 | 8,8 |
| Trouvé | 65,31 | 5,59 | 14,98 | 8,52 |

Spectre IR (KBr) :
- bandes NH à 3300 et 3220 $cm^{-1}$
- bandes CH à 3120, 3090, 2980, 2930 et 2820 $cm^{-1}$
- bandes CO à 1495, 1445, 1305, 1050, 770 et 730 $cm^{-1}$,

à partir d'hydroxy-6 dihydro-5,6 oxo-4 4H-pyrrolo[1,2-a] thiéno[2,3-f] diazépine[1,4] et de N-phénylpipérazine, avec un rendement de 76 %.

**19) BENZYLAMINO-6 DIHYDRO-5, 6 OXO-4 4H-PYRROLO [1,2-a] THIENO (2,3-f] DIAZEPINE [1,4]** sous forme de cristaux blancs fondant à 161° C (éther éthylique):

Analyse élémentaire :

| | C % | H % | N % | S % |
|---|---|---|---|---|
| Calculé | 66 | 4,89 | 13,58 | 10,36 |
| Trouvé | 65,85 | 5,56 | 13,27 | 10,27 |

Spectre IR (KBr) :
- bandes NH à 3340 et 3200 $cm^{-1}$
- bandes CH à 3080 et 2900 $cm^{-1}$
- bandes principales à 1550, 1500, 1460, 1130 et 720 $cm^{-1}$

à partir d'hydroxy-6 dihydro-5,6 oxo-4 4H-pyrrolo [1,2-a] thiéno[2,3-f] diazépine[1,4] et de benzylamine avec un rendement de 72 %

**EXEMPLE 20 :**

**DIHYDRO-5,6 OXO-4 4H-PYRROLO[1,2-a] THIENO[2,3-f]DIAZEPINE[1,4] :**

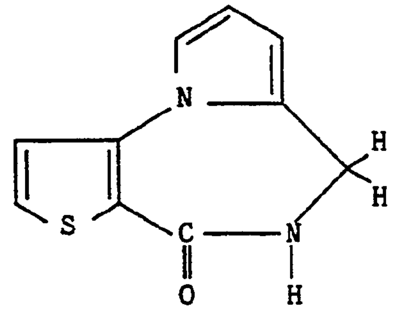

A une solution de 2 g (0,007 mole) de pipéridino-6 dihydro-5,6 oxo-4 4H-pyrrolo[1,2-a] thiéno[2,3-f] diazépine[1,4] dans 120 ml de méthanol, est ajouté, par petites fractions, 1 g (0,0028 mole) de borohydrure de sodium et le mélange réactionnel est agité à température ambiante pendant 15 minutes, puis chauffé au reflux pendant 30 minutes. Le méthanol est ensuite éliminé sous vide et le résidu solide trituré dans 200 ml d'eau. Le précipité

est essoré, lavé à l'eau, séché et recristallisé.

On obtient 1,2 g de dihydro-5,6 oxo-4 4H-pyrrolo[1,2-a] thiéno[2,3-f] diazépine[1,4], sous forme de cristaux blancs fondant à 191°C (éther éthylique).

Rendement : 84 %

Analyse élémentaire :

| | C % | H % | N % | S % |
|---|---|---|---|---|
| Calculé | 58,81 | 3,95 | 13,72 | 15,70 |
| Trouvé | 58,70 | 3,97 | 13,64 | 15,54 |

Spectre IR (KBr) :
- bandes NH à 3230 et 3160 $cm^{-1}$
- bandes CH à 3110, 3030 et 2890 $cm^{-1}$
- bande CO à 1635 $cm^{-1}$
- bandes principales à 1540, 1490, 1450, 1330, 1195, 790, 755 et 730 $cm^{-1}$.

**EXEMPLE 21 :**

**ACETONYL-6 DIHYDRO-5, 6 OXO-4 4H-PYRROLO [1,2-a] THIENO [2,3-f] DIAZEPINE [1,4] :**

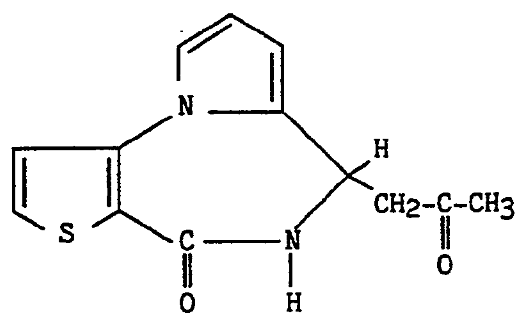

1 g (0,0045 mole) d'hydroxy-6 dihydro-5,6 oxo-4 4H-pyrrolo[1,2-a] thiéno[2,3-f] diazépine[1,4] est ajouté en une fois à une émulsion de 20 ml d'acétone dans 10 ml de soude 10N, et le mélange réactionnel est agité fortement à température ambiante pendant 3 heures. L'acétone est alors éliminée sous vide et 100 ml d'eau sont versés sur le résidu liquide. Le précipité jaune obtenu est essoré, lavé à l'eau et recristallisé.

On obtient 0,7 g d'acétonyl-6 dihydro-5,6 oxo-4 4H-pyrrolo[1,2-a] thiéno[2,3-f] diazépine[1,4], sous forme de cristaux blancs fondant à 268°C (isopropanol).

Rendement : 60 %

Analyse élémentaire :

| | C % | H % | N % | S % |
|---|---|---|---|---|
| Calculé | 59,98 | 4,65 | 10,76 | 12,32 |
| Trouvé | 59,78 | 4,72 | 10,64 | 11,96 |

Spectre IR (KBr) :
- bandes NH à 3270 et 3150 $cm^{-1}$
- bandes CH à 3040, 2980 et 2880 $cm^{-1}$

– bandes CO à 1700 $cm^{-1}$ (cétone) et 1640 $cm^{-1}$ (lactame)
– bandes principales à 1540, 1490, 1455, 1380, 1140, 765 et 720 $cm^{-1}$.

EXEMPLES 22 A 24

En opérant de façon analogue à celle décrite dans l'exemple 21, ont été préparés les dérivés suivants :

**22) (METHYL-3 OXO-2 BUTYL-1)-6 DIHYDRO-5,6 OXO-4 4H-PYRROLO [1,2-a] THIENO[2,3-f] DIAZEPINE[1,4]** sous forme de cristaux blancs fondant à 172° C (éther éthylique)

Analyse élémentaire :

| | C % | H % | N % | S % |
|---|---|---|---|---|
| Calculé | 62,48 | 5,59 | 9,71 | 11,12 |
| Trouvé | 62,39 | 5,44 | 9,88 | 10,89 |

Spectre IR (KBr) :
– bandes NH à 3290 et 3130 $cm^{-1}$
– bandes CH à 3140, 3100, 3000 et 2950 $cm^{-1}$
– bandes C=O à 1715 $cm^{-1}$ (éther) et 1645 $cm^{-1}$ (lactame)
– bandes principales à 1500, 1340, 1170, 960, 870 et 745 $cm^{-1}$.

à partir d'hydroxy-6 dihydro-5,6 oxo-4 4H-pyrrolo [1,2-a] thiéno[2,3-f] diazépine[1,4] et d'isopropylméthylcétone avec un rendement de 16 %

**23) PHENACYL-6 DIHYDRO-5,6 OXO-4 4H-PYRROLO [1,2-a] THIENO[2,3f] DIAZEPINE[1,4]** sous forme de cristaux blances fondant à 214° C (éther éthylique)

Analyse élémentaire :

| | C % | H % | N % | S % |
|---|---|---|---|---|
| Calculé | 67,06 | 4,38 | 8,69 | 9,94 |
| Trouvé | 66,99 | 4,44 | 8,69 | 10,20 |

Spectre IR (KBr) :
– bandes NH à 3280 et 3170 $cm^{-1}$
– bandes CH à 2960 et 2830 $cm^{-1}$
– bandes C=O à 1690 $cm^{-1}$ (cétone) et 1655 $cm^{-1}$ (lactame)
– bandes principales à 1490, 1450, 1140, 770 et 695 $cm^{-1}$.

à partir d'hydroxy-6 dihydro-5,6 oxo-4 4H-pyrrolo[1,2-a] thiéno[2,3-f] diazépine[1,4] et d'acétophénone avec un rendement de 15 %

**24) (FLUORO-4' PHENACYL)-6 DIHYDRO-5,6 OXO-4 4H-PYRROLO [1,2-a] THIENO[2,3-f] DIAZEPINE[1,4]** sous forme de cristaux jaunes fondant à 250° C (éther éthylique)

Analyse élémentaire :

| | C % | H % | N % | S % | F % |
|---|---|---|---|---|---|
| Calculé | 63,51 | 3,84 | 8,23 | 9,41 | 5,58 |
| Trouvé | 65,98 | 3,65 | 8,15 | 9,62 | 5,39 |

Spectre IR (KBr) :
- bandes NH à 3280 et 3180 $cm^{-1}$
- bandes CH à 3000, 2900 et 2840 $cm^{-1}$
- bandes C=O à 1670 $cm^{-1}$ (éther) et 1630 $cm^{-1}$ (lactame)
- bandes principales à 1480, 1435, 1140, 1110, 760 et 710 $cm^{-1}$.

à partir d'hydroxy-6 dihydro-5,6 oxo-4 4H-pyrrolo[1,2-a] thiéno[2,3-f] diazépine[1,4] et de parafluoroacétophénone avec un rendement de 16 %

**EXEMPLE 25 :**

**(HYDROXY-2 PROPYL)-6 DIHYDRO-5,6 OXO-4 4H-PYRROLO[1,2-a] THIENO[2,3-f] DIAZEPINE[1,4] :**

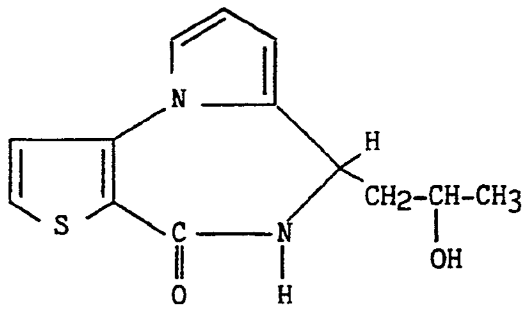

A une solution de 1 g (0,0038 mole) d'acétonyl-6 dihydro-5,6 oxo-4 4H-pyrrolo[1,2-a] thiéno[2,3-f] diazépine[1,4] dans 250 ml de méthanol, on ajoute 0,58 g (0,0152 mole) de borohydrure de sodium, puis le mélange réactionnel est agité à température ambiante pendant 3 heures. Le méthanol est ensuite éliminé sous vide et le résidu solide est trituré dans 200 ml d'eau. Le précipité obtenu est essoré, lavé à l'eau, séché et recristallisé.

On obtient 0,8 g d'(hydroxy-2 propyl)-6 dihydro-5,6 oxo-4 4H-pyrrolo[1,2-a] thiéno[2,3-f] diazépine[1,4], sous forme de cristaux blancs fondant à 170°C (éther éthylique).

Rendement : 80 %

Analyse élémentaire :

| | C % | H % | N % | S % |
|---|---|---|---|---|
| Calculé | 59,52 | 5,38 | 10,68 | 12,22 |
| Trouvé | 59,55 | 5,18 | 10,58 | 12,06 |

Spectre IR (KBr) :
- bande OH à 3420 $cm^{-1}$
- bandes NH à 3360 et 3180 $cm^{-1}$
- bandes CH à 3030, 2970 et 2860 $cm^{-1}$
- bande CO à 1625 $cm^{-1}$
- bandes principales à 1485, 1440, 870, 765 et 710 $cm^{-1}$.

**EXEMPLE 26 :**

**(ETHOXYCARBONYL-CYANO-METHYL)-6 DIHYDRO-5,6 OXO-4 4H-PYRROLO [1,2-a] THIENO[2,3-f] DIAZEPINE[1,4] :**

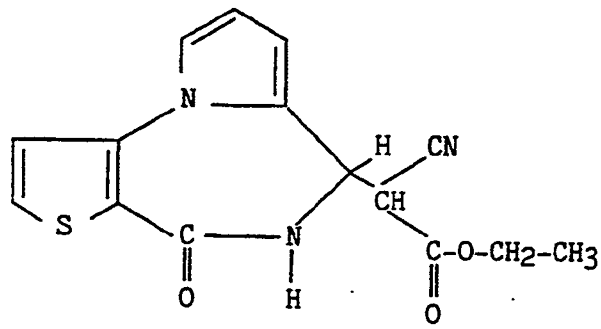

A une solution de 1 g (0,0045 mole) d'hydroxy-6 dihydro-5,6 oxo-4 4H-pyrrolo[1,2-a] thiéno[2,3-f] diazépine[1,4] dans 100 ml d'acétonitrile sont ajoutés 2 ml de cyanoacétate d'éthyle et 0,5 ml de triéthylamine, puis le mélange réactionnel est agité à température ambiante pendant 12 heures. L'acétonitrile est ensuite éliminé sous vide. Le résidu obtenu est trituré dans 200 ml d'eau. Le précipité formé est essoré, lavé à l'eau, séché et recristallisé.

On obtient 0,7 g d'(éthoxycarbonyl-cyano-méthyl)-6 dihydro-5,6 oxo-4 4H-pyrrolo[1,2-a] thiéno[2,3-f] diazépine[1,4], sous forme de cristaux jaunes fondant à 195°C (éther éthylique).

Rendement : 49 %

Analyse élémentaire :

| | C % | H % | N % | S % |
|---|---|---|---|---|
| Calculé | 57,13 | 4,16 | 13,33 | 10,17 |
| Trouvé | 57,07 | 4,24 | 13,28 | 10,06 |

Spectre IR (KBr) :
- bandes NH à 3340, 3280 et 3180 $cm^{-1}$
- bandes CH à 3120 et 2990 $cm^{-1}$
- bande C ≡ N à 2230 $cm^{-1}$
- bandes CO à 1720 $cm^{-1}$ (éther) et 1670 $cm^{-1}$ (lactame)
- bandes principales à 1595, 1465, 1435, 1365, 1300, 1240, 1090, 875 et 750 $cm^{-1}$.

**EXEMPLE 27 :**

**(METHOXYCARBONYL-CYANO-METHYL)-6 DIHYDRO-5,6 OXO-4 4H-PYRROLO[1,2-a] THIENO[2,3-f] DIAZEPINE[1,4]**, préparé comme décrit dans l'exemple 26 à partir d'hydroxy-6 dihydro-5, 6 oxo-4 4H-pyrrolo [1,2-a] thiéno [2,3-f] diazépine[1,4] et de cyanoacétate de méthyle.

**EXEMPLE 28 :**

**(ETHOXYCARBONYL-1 ACETONYL)-6 DIHYDRO-5,6 OXO-4 4H-PYRROLO[1,2-a] THIENO[2,3-f] DIAZEPINE[1,4] :**

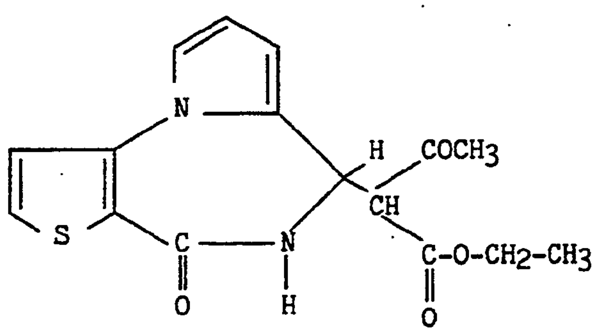

En opérant comme dans l'exemple 26, à partir de 1 g d'hydroxy-6 dihydro-5,6 oxo-4 4H-pyrrolo[1,2-a] thiéno[2,3-f] diazépine[1,4] et de 2 ml d'acétoacétate d'éthyle, ont été obtenus 0,7 g d'(éthoxycarbonyl-1 acétonyl)-6 dihydro-5,6 oxo-4 4H-pyrrolo[1,2-a] thiéno[2,3-f] diazépine[1,4], sous forme de cristaux blancs fondant à 196°C (éther éthylique).

Rendement : 47 %

Analyse élémentaire :

| | C % | H % | N % | S % |
|---|---|---|---|---|
| Calculé | 57,82 | 4,85 | 8,43 | 9,65 |
| Trouvé | 57,75 | 4,78 | 8,37 | 9,85 |

Spectre IR (KBr) :
- bandes NH à 3270 et 3200 $cm^{-1}$
- bandes CH à 3120, 2940 et 2870 $cm^{-1}$
- bandes CO à 1710 et 1635 $cm^{-1}$
- bandes principales à 1545, 1495, 1300, 1255, 1030 et 790 $cm^{-1}$.

EXEMPLE 29 :

**DIHYDRO-5,6 4H-PYRROLO[1,2-a] THIENO[2,3-f] DIAZEPINE[1,4] ET SON CHLORHYDRATE :**

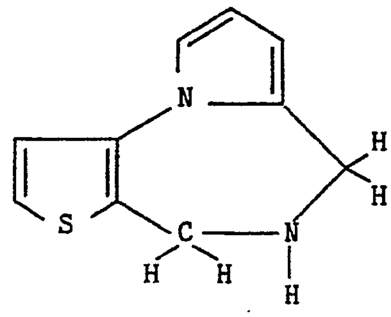

Une solution de 5,3 g (0,024 mole) d'hydroxy-6 dihydro-5,6 oxo-4 4H-pyrrolo[1,2-a] thiéno[2,3-f] diazépine[1,4] dans 400 ml de dichlorométhane est additionnée par petites fractions à une suspension de 3,66 g (0,096 mole) d'hydrure d'aluminium et de lithium dans 20 ml d'éther éthylique. Le mélange réactionnel est agité à température ambiante pendant 30 minutes puis chauffé au reflux pendant 6 heures. Après refroidissement, la solution obtenue est versée sur 250 g de glace. L'émulsion formée est essorée puis la phase organique est décantée. La phase aqueuse est extraite par 200 ml de dichlorométhane. Les phases organiques sont rassemblées, séchées sur chlorure de calcium, purifiées au charbon animal et évaporées sous vide. L'huile résiduelle est mise en solution dans 300 ml d'éther éthylique et on fait barbotter, dans cette solution éthérée, pendant 15 secondes, un courant d'HCl gazeux. Le précipité est essoré, lavé à l'éther et recristallisé.

On obtient ainsi 3,2 g de chlorhydrate de dihydro-5,6 4H-pyrrolo[1,2-a] thiéno[2,3-f] diazépine[1,4] sous forme de cristaux beiges fondant à 260°C (isopropanol).

Rendement : 59 %

Analyse élémentaire :

| | C % | H % | N % | Cl % |
|---|---|---|---|---|
| Calculé | 52,98 | 4,89 | 12,36 | 15,64 |
| Trouvé | 53,20 | 4,98 | 13,93 | 15,46 |

Spectre IR (KBr) :
- bandes CH à 3100 et 3080 $cm^{-1}$
- bande large ($NH_2^+Cl^-$) de 2900 à 2470 $cm^{-1}$
- bandes principales à 1565, 1550, 1480, 1445, 1296, 1190, 1066, 740 et 720 $cm^{-1}$.

EXEMPLE 30 :

**p.TOLYLSUFONYL-5 DIHYDRO-5,6 4H-PYRROLO [1,2-a] THIENO[2,3-f] DIAZEPINE[1,4] :**

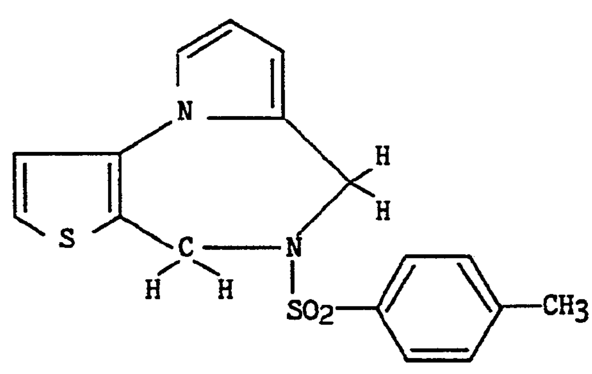

A une solution de 1 g (0,0053 mole) de dihydro-5,6 4H-pyrrolo[1,2-a] thiéno[2,3-f] diazépine[1,4] dans 10 ml de pyridine, on ajoute un excès de chlorure de paratoluène sulfonyle puis le mélange réactionnel est agité à température ambiante pendant une heure. La pyridine est alors éliminée sous pression réduite et le résidu huileux obtenu est trituré dans 100 ml d'eau. L'émulsion formée est extraite par 3 fois 100 ml d'éther éthylique. Les phases organiques sont décantées, rassemblées, séchées, lavées à l'eau acidulée, séchées sur sulfate de magnésium et évaporées sous vide. Le résidu solide est recristallisé.

On obtient ainsi 0,4 g de p.tolylsulfonyl-5 dihydro-5,6 4H-pyrrolo[1,2-a] thiéno[2,3-f] diazépine[1,4], sous forme de cristaux jaunes fondant à 165°C (éther éthylique).

Rendement : 22 %

Analyse élémentaire :

| | C % | H % | N % | S % |
|---|---|---|---|---|
| Calculé | 59,28 | 4,68 | 8,13 | 18,62 |
| Trouvé | 59,21 | 4,72 | 8,05 | 18,44 |

Spectre IR (KBr) :
- bandes CH à 3100 et 2830 $cm^{-1}$
- bandes principales à 1580, 1490, 1440, 1355, 1165, 1090, 730 et 660 $cm^{-1}$.

**EXEMPLE 31 :**

**ACETYL-5 DIHYDRO-5,6 OXO-4 4H-PYRROLO[1,2-a] THIENO[2,3-f] DIAZEPINE[1,4] :**

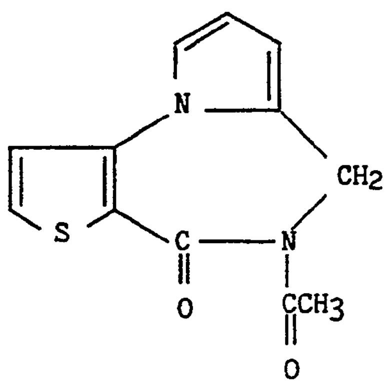

Une suspension de 0,5 g de dihydro-5,6 oxo-4 4H-pyrrolo[1,2-a] thiéno[2,3-f] diazépine[1,4] (exemple 20) dans 20 ml d'anhydride acétique est chauffée au reflux pendant 2 heures. L'anhydride acétique est ensuite éliminé sous vide. Le résidu huileux est trituré dans 100 ml d'eau et l'émulsion obtenue extraite à l'éther éthylique. Les phases éthérées sont séchées sur sulfate de magnésium et concentrées sous pression partielle. L'huile résiduelle qui cristallise par adition d'éther de pétrole est recristallisée.

On obtient ainsi 0,4 g (66%) d'acétyl-5 dihydro-5,6 oxo-4 4H-pyrrolo[1,2-a] thiéno[2,3-f] diazépine[1,4] sous forme de cristaux jaunes fondant à 157° C (éther éthylique)

Analyse élémentaire :

| | C % | H % | N % | S % |
|---|---|---|---|---|
| Calculé | 58,48 | 4,06 | 5,69 | 12,99 |
| Trouvé | 58,48 | 5,72 | 5,72 | 13,05 |

Spectre IR (KBr) :
- bandes CH à 3150, 3120 et 3100 $cm^{-1}$

– bandes C=O à 1695 et 1660 $cm^{-1}$
– bandes principales à 1540, 1490, 1430, 1345, 1220, 1200, 1150, 770 et 725 $cm^{-1}$.

**EXEMPLE 32 :**

**(N-PHENYL CARBAMOYLE)-5 DIHYDRO-5,6 OXO-4 4H-PYRROLO[1,2-a] THIENO[2,3-f] DIAZEPINE[1,4] :**

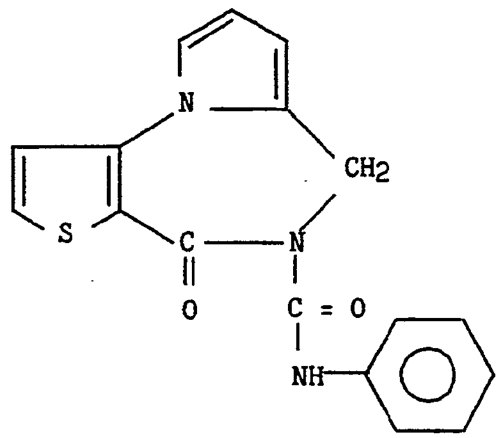

Une solution de 1 g de dihydro-5,6 oxo-4 4H-pyrrolo[1,2-a] thiéno[2,3-f] diazépine[1,4] (exemple 20), 0,5 ml d'isocyanate de phényle et 1,36 ml de triéthylamine dans 200 ml de toluène est chauffée au reflux pendant 2 heures. Le milieu réactionnel est ensuite concentré sous pression réduite puis repris à l'eau et extrait à l'éther éthylique.

Les phases éthérées sont séchées puis concentrées sous pression réduite.

On obtient 1 g (63 %) de (N-phényl carbamoyle)-5 dihydro-5,6 oxo-4 4H-pyrrolo[1,2-a] thiéno[2,3-f] diazépine[1,4] sous forme de cristaux blancs fondant à 176° C (éther éthylique)

Analyse élémentaire :

| | C % | H % | N % | S % |
|---|---|---|---|---|
| Calculé | 63,14 | 4,05 | 12,99 | 9,91 |
| Trouvé | 63,07 | 4,08 | 13,07 | 9,92 |

Spectre IR (KBr) :
– bandes NH à 3260 et 3220 $cm^{-1}$
– bande CH à 3120 $cm^{-1}$
– bande C=O à 1710 $cm^{-1}$
– bandes principales à 1590, 1550, 1490, 1430, 1195, 770 et 715 $cm^{-1}$.

**EXEMPLE 33 :**

**(N-PROPYL CARBAMOYLE)-5 DIHYDRO-5,6 OXO-4 4H-PYRROLO[1,2-a] THIENO[2,3-f] DIAZEPINE[1,4] :**

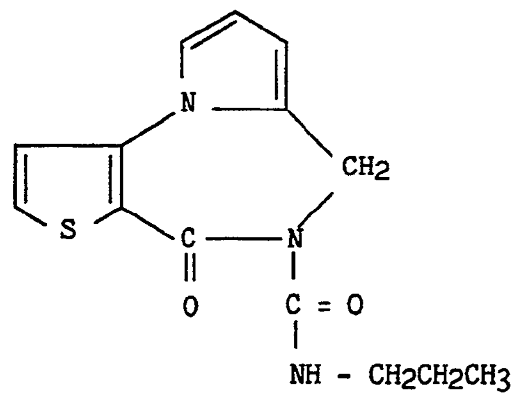

On procède comme pour l'exemple 32 en remplaçant l'isocyanate de phényle par l'isocyanate de n propyle

On obtient le (N-propyl carbamoyle)-5 dihydro-5,6 oxo-4 4H-pyrro1o[1,2-a] thiéno[2,3-f] diazépine[1,4] sous forme de cristaux blancs fondant à 120° C (éther éthylique) avec un rendement de 63 %

Analyse élémentaire :

| | C % | H % | N % | S % |
|---|---|---|---|---|
| Calculé | 58,11 | 5,22 | 14,52 | 11,08 |
| Trouvé | 58,15 | 5,19 | 14,48 | 11,16 |

Spectre IR (KBr) :
- bande NH à 3290 $cm^{-1}$
- bandes CH à 3070, 3030, 2970 et 2880 $cm^{-1}$
- bandes C=O à 1690 et 1625 $cm^{-1}$
- bandes principales à 1485, 1430, 1380, 1330, 1200, 1210, 775 et 700 $cm^{-1}$.

Les paramètres RMN relatifs aux dérivés objet des exemples 1 à 33 sont regroupés dans les tableaux ci après.

**TABLEAU I**

Paramètres RMN relatifs aux dérivés des exemples 1 à 6 correspondant aux formules Ia et Ib regroupées dans la formule suivante :

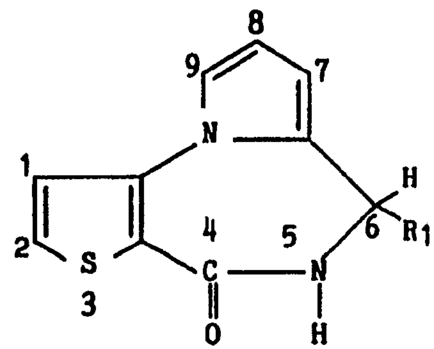

| Exemples N° | $R_1$ | Solvant | $H_1$ | $H_2$ | $H_7$ | $H_8$ | $H_9$ | NH | $H_6$ | Autres Protons |
|---|---|---|---|---|---|---|---|---|---|---|
| 1 | OH | DMSO | 7,21 | 7,21 | 6,25 | 6,25 | 7,17 | 8,59 | 5,59 | OH : 6,25 |
| 2 | $OCH_3$ | $CDCl_3$ | 7,20 | 7,56 | 6,30 | 6,30 | 7,10 | 7,47 | 5,35 | $CH_3$ : 3,30 |
| 3 | $OCH_2CH_3$ | $CDCl_3$ | 7,18 | 7,57 | 6,29 | 6,29 | 7,08 | 7,43 | 5,46 | $CH_2$ : CH : 3,70<br>CH : 3,46<br>$CH_3$ : 1,08 |
| 4 | $OCH_2CH_2CH_3$ | $CDCl_3$ | 7,17 | 7,56 | 6,28 | 6,28 | 7,07 | 7,9 | 5,48 | $CH_2$ : 3,52<br>3,34<br>$CH_2$ : 1,45<br>$CH_3$ : 0,71 |
| 5 | 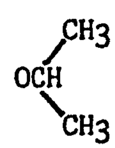 | $CDCl_3$ | 7,17 | 7,56 | 6,30 | 6,25 | 7,07 | 7,38 | 5,56 | CH : 3,81<br>$2CH_3$ : 1,07 |
| 6 | $OCH_2C_6H_5$ | $CDCl_3$ | 7,17 | 7,58 | 6,29 | 6,22 | 7,08 | 7,42 | 5,47 | $CH_2$ : 4,52<br>$C_6H_5$ : 5,24 |

**TABLEAU II**

Paramètres RMN relatifs aux dérivés des exemples 7 à 9 de formule Ic :

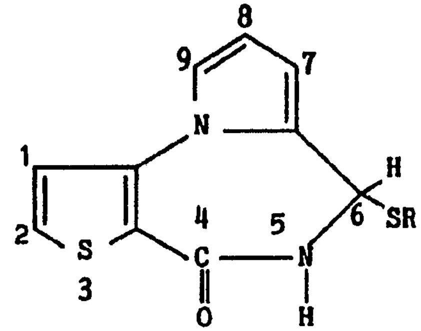

| Exemples N° | R | Solvant | $H_1$ | $H_2$ | $H_7$ | $H_8$ | $H_9$ | $H_6$ | NH | Autres Protons |
|---|---|---|---|---|---|---|---|---|---|---|
| 7 | $CH_2COOCH_3$ | $CDCl_3$ | 7,18 | 7,62 | 6,26 | 6,30 | 7,10 | 5,99 | 6,94 | CH : CH : 3,34<br>CH : 3,04<br>$CH_3$ : 3,77 |
| 8 | $C_6H_5$ | DMSO | 7,50 | 7,94 | 6,28 | 6,28 | 7,42 | 5,78 | 9,13 | $C_6H_5$ : 7,31 et 7,32 |
| 9 | $CH_2CH_2COOH$ | $CDCl_3$ | 7,21 | 7,65 | 6,14 | 6,24 | 7,09 | 5,79 | 6,75 | OH : 12,19<br>$CH_2$ : 2,60<br>$CH_2$ : 2,43 |

## TABLEAU III

Paramètres RMN relatifs aux dérivés des exemples 10 à 19 de formule Id :

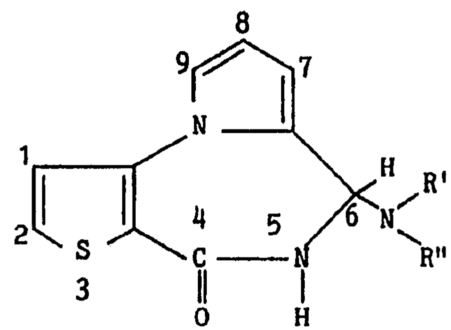

| Exemples N° | R' | R" | Solvant | $H_1$ | $H_2$ | $H_7$ | $H_8$ | $H_9$ | NH | $H_6$ | Autres Protons |
|---|---|---|---|---|---|---|---|---|---|---|---|
| 10 | H | $CH_3$ | DMSO | 7,59 | 7,84 | 6,21 | 6,21 | 7,32 | 8,63 | 4,86 | $CH_3$ : 2,20 |
| 11 | H | $CH_2CH_3$ | $CDCl_3$ | 7,17 | 7,59 | 6,18 | 6,31 | 7,03 | 6,71 | 5,15 | NH : 1,68<br>$CH_2$ : CH : 2,87<br>CH : 2,71<br>$CH_3$ : 1,09 |
| 12 | H | $CH_2CH_2CH_3$ | $CDCl_3$ | 7,16 | 7,58 | 6,18 | 6,31 | 7,03 | 6,67 | 5,15 | NH : 1,66<br>$CH_2$ :2,68 et 1,47<br>$CH_3$ : 0,87 |
| 13 | 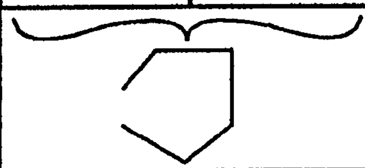 | | $CDCl_3$ | 7,16 | 7,52 | 6,18 | 6,27 | 7,03 | 6,72 | 4,61 | $CH_2$ : 2,63 ; 2,27<br>$2CH_2$ :1,63 |
| 14 | 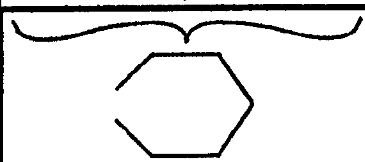 | | $CDCl_3$ | 7,13 | 7,5 | 6,17 | 6,28 | 7,02 | 7,36 | 4,64 | $CH_2$ : 2,56 et<br>2,15<br>$3CH_2$ : 1,35 |

| Exemples N° | R' | R" | Solvant | $H_1$ | $H_2$ | $H_7$ | $H_8$ | $H_9$ | NH | $H_6$ | Autres Protons |
|---|---|---|---|---|---|---|---|---|---|---|---|
| 15 | 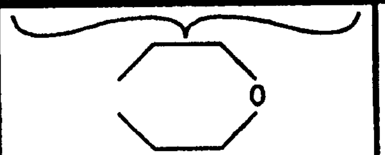 | | $CDCl_3$ | 7,14 | 7,54 | 6,22 | 6,29 | 7,06 | 7,11 | 4,56 | $2CH_2$ : 3,47<br>$CH_2$ : 2,56 et 2,10 |
| 16 | H | 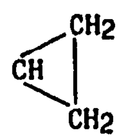 | DMSO | 7,42 | 7,84 | 6,20 | 6,20 | 7,30 | 8,67 | 5,04 | NH : 2,83<br>CH : 2,09<br>$2CH_2$ : 0,26 |
| 17 | 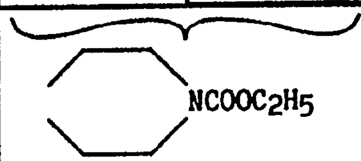 | | $CDCl_3$ | 7,14 | 7,53 | 6,21 | 6,30 | 7,06 | 7,23 | 4,60 | $CH_2$ : 4,07 ; 2,48 et 2,06<br>$2CH_2$ : 3,24 |
| 18 | 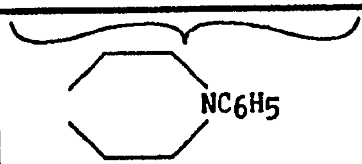 | | DMSO | 7,14 | 7,50 | 6,25 | 6,30 | 7,13 | 9,01 | 5,64 | $2CH_2$ : 3,02<br>$2CH_2$ : 2,83<br>$C_6H_5$ :7,26 ; 6,84 |
| 19 | H | 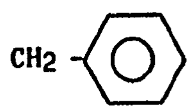 | DMSO | 7,46 | 7,88 | 6,16 | 6,25 | 7,35 | 8,80 | 4,99 | NH : 2,83<br>$CH_2$ : 3,70<br>$C_6H_5$ :7,44 ; 7,35 |

## TABLEAU IV

Paramètres RMN relatifs au dérivé de l'exemple 20 de formule Ie :

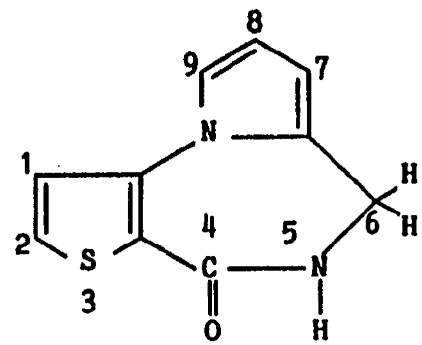

| Exemple N° | Solvant | $H_1$ | $H_2$ | $H_7$ | $H_8$ | $H_9$ | $CH_2$ 6 | Autres Protons |
|---|---|---|---|---|---|---|---|---|
| 20 | $CDCl_3$ | 7,16 | 7,60 | 6,12 | 6,29 | 7,01 | 4,32 | NH : 6,91 |

TABLEAU V

Paramètres RMN relatifs aux dérivés des exemples 21 à 28 de formules If à Il regroupés dans la formule suivante :

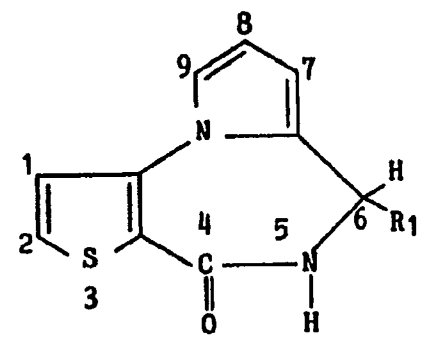

| Exemples N° | $R_1$ | Solvant | $H_1$ | $H_2$ | $H_7$ | $H_8$ | $H_9$ | NH | $H_6$ | Autres Protons |
|---|---|---|---|---|---|---|---|---|---|---|
| 21 | $-CH_2COCH_3$ | DMSO | 7,45 | 7,96 | 6,08 | 6,24 | 7,30 | 8,34 | 3,68 | $CH_2$ : 3,16<br>$CH_3$ : 2,17 |
| 22 | 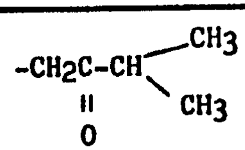 | $CDCl_3$ | 7,86 | 7,41 | 6,06 | 6,23 | 7,27 | 8,26 | 4,6 | $CH_2$ : 3,12<br>CH : 3,12<br>2 $CH_2$ : 1,12 |
| 23 | 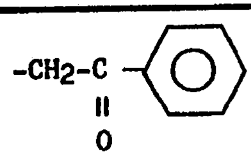 | DMSO | 7,89 | 7,42 | 6,15 | 6,15 | 7,25 | 8,35 | 4,8 | $CH_2$ : 3,35<br>$C_6H_5$ :7,94 et 7,42 |
| 24 | 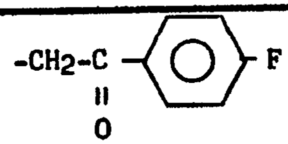 | DMSO | 7,82 | 7,39 | 6,12 | 6,25 | 7,26 | 8,61 | 4,78 | $CH_2$ : 3,39<br>$C_6H_5$ :7,12 et 7,04 |
| 25 | $-CH_2CHOHCH_3$ | $CDCl_3$ | 7,14 | 7,58 | 6,08 | 6,30 | 7,01 | 7,69 | 4,70 | CH : 4,24<br>$CH_2$ : 2,18<br>$CH_3$ : 1,30 |

| Exemples N° | $R_1$ | Solvant | $H_1$ | $H_2$ | $H_7$ | $H_8$ | $H_9$ | NH | $H_6$ | Autres Protons |
|---|---|---|---|---|---|---|---|---|---|---|
| 26 | 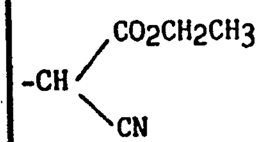 | $CDCl_3$ | 7,69 | 7,69 | 7,20 | 7,20 | 7,69 | 7,69 | 5,40 | $CH_2$ : 4,28<br>CH : 5,04<br>$CH_3$ : 1,38 |
| 27 | 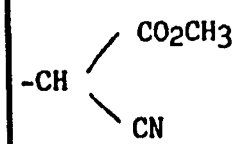 | DMSO | 7,66 | 7,92 | 6,24 | 6,24 | 7,66 | 7,66 | 6,64 | $CH_3$ : 3,75 |
| 28 | 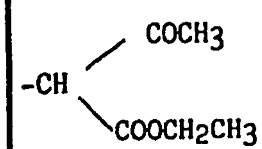 | $CDCl_3$ | 7,20 | 7,65 | 6,11 | 6,28 | 7,04 | 6,62 | 5,16 | $CH_2$ : 4,25<br>CH : 4,13<br>$CH_3$ : 1,29<br>$CH_3$ : 1,91 |

## TABLEAU VI

Exemple 25 :

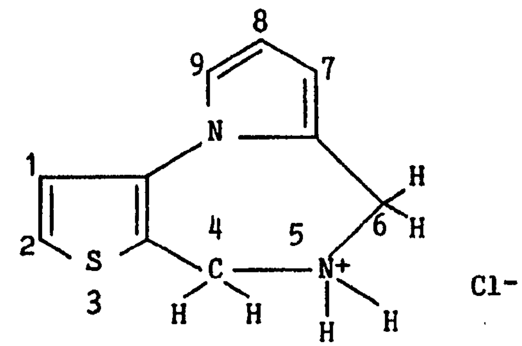

Solvant DMSO

| $H_1$ | $H_2$ | $H_7$ | $H_8$ | $H_9$ | $NH_2$ | $CH_2$ 4 | $CH_2$ 6 |
|---|---|---|---|---|---|---|---|
| 7,40 | 7,77 | 6,43 | 6,27 | 7,33 | 9,95 | 4,17 | 4,09 |

## TABLEAU VII

Exemple 26 :

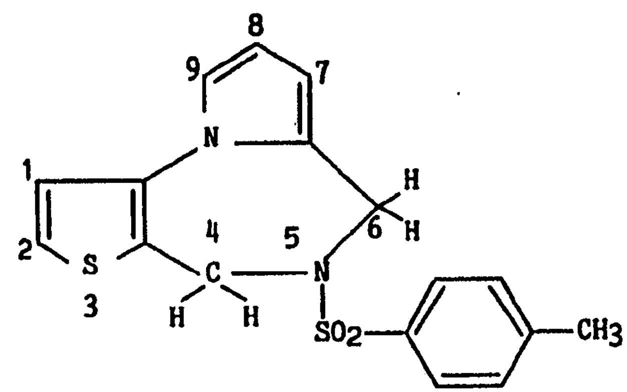

Solvant : $CDCl_3$

| $H_1$ | $H_2$ | $H_7$ | $H_8$ | $H_9$ | $CH_2$ 4 | $CH_2$ 6 | Autres Protons |
|---|---|---|---|---|---|---|---|
| 6,98 | 7,19 | 6,14 | 6,14 | 6,82 | 4,47 | 4,39 | $C_6H_4$ : 7,65 et 7,23<br>$CH_3$ : 2,38 |

TABLEAU VIII

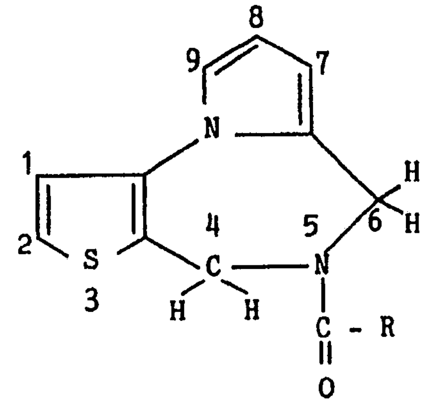

| Exemples N° | R | Solvant | $H_1$ | $H_2$ | $H_7$ | $H_8$ | $H_9$ | $H_6$ | Autres Protons |
|---|---|---|---|---|---|---|---|---|---|
| 31 | $-CH_3$ | DMSO | 7,56 | 8,19 | 6,26 | 6,26 | 7,43 | 5,82 | $CH_3$ : 2,36 |
| 32 | -NH – 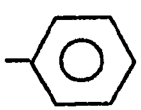 | DMSO | 7,56 | 8,21 | 6,32 | 6,32 | 7,52 | 5,79 | $C_6H_5$:7,55 et 7,36 |
| 33 | $-NH-CH_2CH_2CH_3$ | DMSO | 7,52 | 8,14 | 6,24 | 6,30 | 7,40 | 4,90 | NH : 8,79<br>$CH_2$ :3,17 et 1,48<br>$CH_3$ : 0,86 |

**Revendications**

**1)** Pyrrolo[1,2-a] thiéno[2,3-f] diazépine [1,4] de formule générale (I):

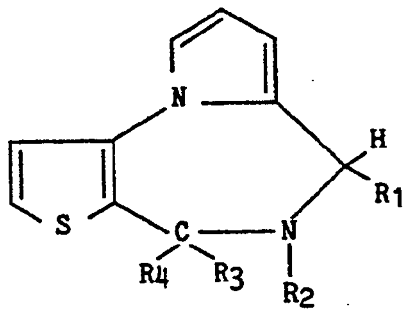

(I)

dans laquelle :
- $R_1$ represente :
  - un atome d'hydrogène,
  - un radical hydroxy,
  - un radical de formule générale :

$$-OR_5 \text{ ou } -SR_5$$

dans laquelle $R_5$ représente :
. un radical alkyle contenant de 1 à 5 atomes de carbone, en chaine droite ou ramifiée, éventuellement substitué par un radical phényle ou par un groupe de formule -COOB dans laquelle B représente un atome d'hydrogène ou un radical alkyle contenant de 1 à 5 atomes de carbone, ou
. un radical aryle tel que par exemple un radical phényle,
  - un radical de formule générale :

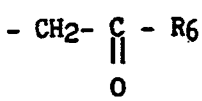 ou 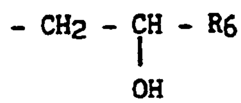

dans laquelle $R_6$ représente :
. un radical alkyle linéaire ou ramifié de 1 à 5 atomes de carbone,
. un radical aryle (éventuellement substitué par un ou plusieurs atomes d'halogènes, groupements alcoxy de 1 à 3 atomes de carbone ou groupements trifluorométhyle ),
. un radical aralkyle de 7 à 9 atomes de carbones (éventuellement substitué sur le noyau aromatique par un ou plusieurs atomes d'halogènes, groupements alcoxy de 1 à 3 atomes de carbone ou groupements trifluorométhyle),
  - un radical de formule :

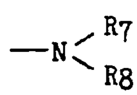

dans laquelle :
. $R_7$ et $R_8$, identiques ou différents, représentent chacun un atome d'hydrogène, un radical alkyle contenant de 1 à 5 atomes de carbone en chaine droite ou ramifiée, un radical cycloalkyle contenant de 3 à 5 atomes de carbone, un radical aryle (éventuellement substitué par un ou plusieurs atomes d'halogènes, groupements alcoxy de 1 à 3 atomes de carbone ou groupements trifluorométhyle) ou un radical aralkyle de 7 à 9 atomes de carbone (éventuellement substitué sur le noyau atomatique par un ou plusieurs atomes d'halogènes, groupements alcoxy de 1 à 3 atomes de carbone ou groupements trifluorométhyle) ou,
. $R_7$ et $R_8$ forment ensemble avec l'atome d'azote auquel ils sont liés un radical heterocyclique penta ou hexagonal renfermant éventuellement un autre hetéroatome tel par exemple un deuxième atome d'azote ou un atome d'oxygène, et éventuellement substitué par un radical alkyle linéaire ou ramifié de 1 à 5 atomes de carbone, par un radical alcoxycarbonyle de 2 à 6 atomes de carbone ou par un radical aryle, aralkyle de 7 à 9 atomes de carbone, hétéroaryle, diaralkyle comme par exemple le radical

benzhydryle (éventuellement substitués sur le ou les noyaux aromatiques par un ou plusieurs substituants identiques ou différents choisis parmi halogène, hydroxy, alkyle ou alcoxy de 1 à 4 atomes de carbone, nitro ou trifluorométhyle),

ou

– un radical alkyle renfermant de 1 à 5 atomes de carbone éventuellement substitué par un ou plusieurs radicaux choisis parmis le groupe formé des radicaux hydroxy, oxo, cyano, alkoxycarbonyle renfermant de 2 à 4 atomes de carbone ;

- $R_2$ représente :

– un atome d'hydrogène,

– un radical alkyle ayant de 1 à 5 atomes de carbone en chaine droite ou ramifiée,

– un radical arylsulfonyle dans lequel le groupe aryle est éventuellement substitué par un radical alkyle ayant de 1 à 5 atomes de carbone en chaine droite ou ramifiée ;

– un radical alkylcarbonyle de 2 à 3 atomes de carbone

– un radical de formule générale :

$$- \underset{\underset{O}{\|}}{C}NHR_9$$

dans laquelle

. $R_9$ représente un radical alkyle linéaire ou ramifié, de 1 à 6 atomes de carbone, un radical aryle ou aralkyle de 7 à 9 atomes de carbone (éventuellement substitués sur le noyau aromatique par un ou plusieurs atomes d'halogène, radicaux alcoxy de 1 à 3 atomes de carbone ou radicaux trifluorométhyle),

- $R_3$ et $R_4$ représentent :

– chacun simultanément un atome d'hydrogène ou,

– ensemble un atome d'oxygène.

**2)** Les sels physiologiquement tolérables des composés de la revendication 1) avec des acides appropriés pharmaceutiquement acceptables

**3)** Les isomères, diastéreoisomères, énantiomères des composés de la revendication 1) isolés ou sous forme de mélange.

**4)** Composé selon la revendication 1) qui est l'hydroxy-6 dihydro-5,6 oxo-4 4H-pyrrolo[1,2-a] thiéno[2,3-f] diazépine[1,4].

**5)** Composé selon la revendication 1) qui est la méthoxy-6 dihydro-5,6 oxo-4 4H-pyrrolo[1,2-a] thiéno[2,3-f] diazépine[1,4].

**6)** Composé selon la revendication 1) qui est la méthoxycarbonylméthylmercapto-6 dihydro-5,6 oxo-4 4H-pyrrolo [1,2-a] thiéno[2,3-f] diazépine[1,4].

**7)** Composé selon la revendication 1) qui est la méthylamino-6 dihydro-5,6 oxo-4 4H-pyrrolo[1,2-a] thiéno[2,3-f] diazepine[1,4] ainsi que ses sels d'addition à un acide minéral ou organique pharmaceutiquement acceptable.

**8)** Composé selon la revendication 1) qui est la dihydro-5,6 oxo-4 4H-pyrrolo[1,2-a] thieno[2,3-f] diazépine[1,4].

**9)** Composé selon la revendication 1) qui est l'acetonyl-6 dihydro-5,6 oxo-4 4H-pyrrolo[1,2-a] thiéno[2,3-f] diazépine[1,4].

**10)** Composé selon la revendication 1) qui est l'(hydroxy-2 propyl)-6 dihydro-5,6 oxo-4 4H-pyrrolo[1,2-a] thiéno[2,3-f] diazépine[1,4].

**11)** Composé selon la revendication 1) qui est l'(éthoxycarbonyl-(cyano)méthyl)-6 dihydro-5,6 oxo-4 4H-pyrrolo[1,2-a] thieno[2,3-f] diazépine[1,4].

**12)** Composé selon la revendication 1) qui est la (méthoxycarbonyl(cyano)méthyl)-6 dihydro-5,6 oxo-4 4H-pyrrolo[1,2-a] thiéno[2,3-f] diazépine[1,4].

**13)** Composé selon la revendication 1) qui est l'(éthoxycarbonyl-1 acétonyl)-6 dihydro-5,6 oxo-4 4H-pyrrolo [1,2-a] thieno[2,3-f] diazépine[1,4].

**14)** Composé selon la revendication 1) qui est la dihydro-5,6 4H-pyrrolo[1,2-a] thieno[2,3-f] diazepine[1,4] ainsi que ses sels d'addition à un acide minéral ou organique pharmaceutiquement acceptable.

**15)** Composé selon la revendication 1) qui est la p.tolylsulfonyl-5 dihydro-5,6 4H-pyrrolo[1,2-a] thiéno[2,3-f] diazépine[1,4].

**16)** Composé selon la revendication 1) qui est l'acétyl-5 dihydro-5,6 oxo-4 4H-pyrrolo[1,2-a] thiéno[2,3-f] diazépine[1,4].

**17)** Composé selon la revendication 1) gui est la (N-phényl carbamoyl)-5 dihydro-5,6 oxo-4 4H-pyrrolo[1,2-a] thiéno[2,3-f] diazépine[1,4].

**18)** Composé selon la revendication 1) qui est la phénacyl-6 dihydro-5,6 oxo-4 4H-pyrrolo[1,2-a] thiéno[2,3-f] diazépine[1,4].

**19)** Composé selon la revendication 1) qui est la morpholino-6 dihydro-5,6 oxo-4 4H-pyrrolo[1,2-a] thiéno[2,3-f] diazepine[1,4], ainsi que ses sels d'addition à un acide minéral ou organique pharmaceutiquement acceptable.

**20)** Composé selon la revendication 1) qui est la pyrrolidino-6 dihydro-5,6 oxo-4 4H-pyrrolo[1,2-a] thiéno[2,3-f] diazépine[1,4], ainsi que ses sels d'addition à un acide minéral ou organique pharmaceutiquement acceptable.

**21)** Procédé de préparation des composés de formule générale **(I)** selon la revendication 1) caractérisé en ce que :

**A** - soit l'on traite l'aminocarbonyl-2 (formyl-2 pyrrolyl-1)-3 thiophène de formule **(II)** :

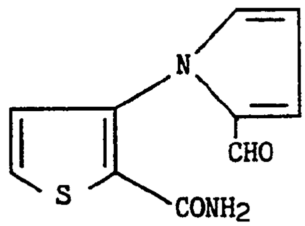

(II)

1) soit avec le mélange eau et triéthylamine, pour donner le composé de formule **(Ia)** :

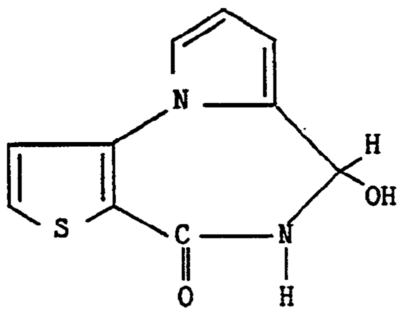

(Ia)

2) soit avec un alcool ou un thiol de formules générales respectives **(III)** et **(IV)** :

$R_5OH$ **(III)**, $R_5SH$ **(IV)**

dans lesquelles $R_5$ a la même signification que dans les composés de formule générale **(I)**, pour donner respectivement les composés de formules générales **(Ib)** et **(Ic)** :

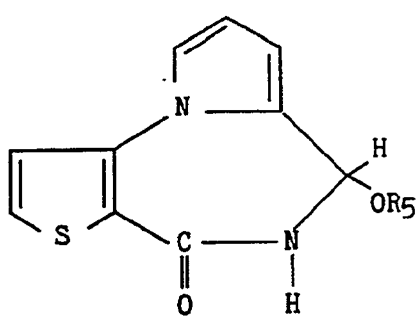

(Ib)

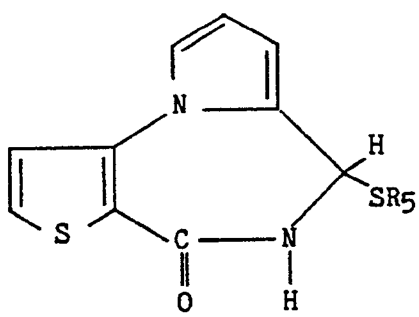

(Ic)

dans lesquelles $R_5$ a la signification précédemment définie ;

3) soit avec une amine secondaire de formule générale **(V)** :

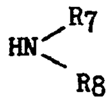

(V)

dans laquelle $R_7$ et $R_8$ ont les mêmes significations que dans les composés de formule générale **(I)**, pour donner les composés de formule générale **(Id)** :

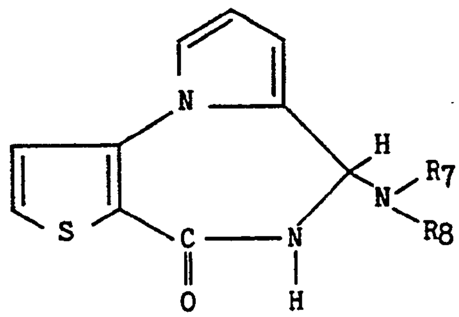

(Id)

dans laquelle $R_7$ et $R_8$ ont les mêmes significations que dans les composés de formule génerale **(I)** ;
4) soit en milieu alacalin avec une méthylcétone de formule générale **(VI)** :

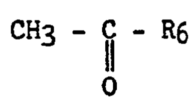

(VI)

dans laquelle $R_6$ a la même signification que dans les composés deformule générale **(I)** de manière à obtenir les composés de formule générale **(If)** :

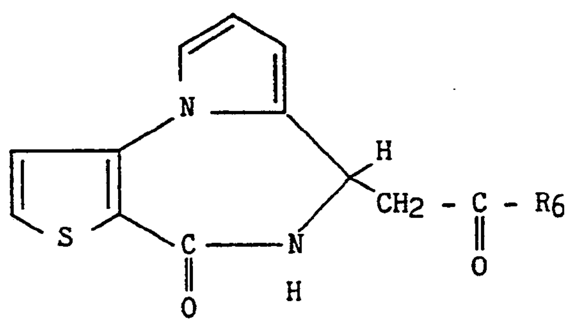

(If)

dans laquelle $R_6$ a la même signification que dans les composés de formule générale **(I)**, que l'on peut éventuellement traiter par un borohydrure de sodium pour obtenir les composés de formule générale **(Ig)** :

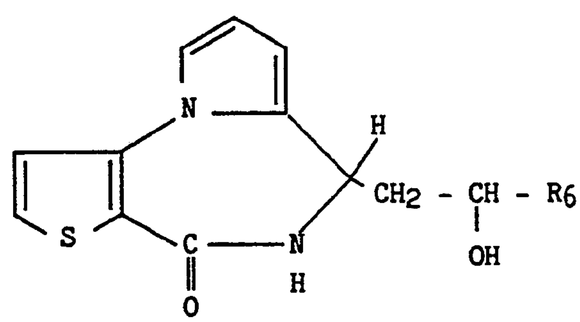

(Ig)

dans laquelle $R_6$ a la même signification que dans les composés de formule générale **(I)** ;
**B** - soit l'on traite le composé de formule générale **(Ia)** précédemment obtenu :

1) soit par un alcool de formule générale **(III)** précédemment définie pour donner les composés de formule générale **(Ib)** :

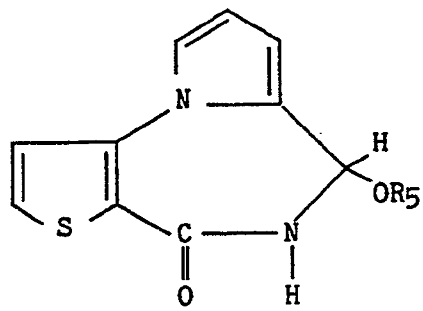

**(Ib)**

dans laquelle $R_5$ a la même définition que dans les composés de formule générale **(I)** ;
2) soit par une amine de formule générale **(V)** précédemment définie pour donner les composés de formule générale **(Id)** :

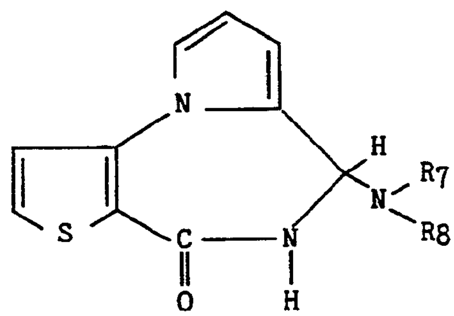

**(Id)**

dans laquelle $R_7$ et $R_8$ ont les mêmes significations que dans les composés de formule générale **(I)**, que l'on peut éventuellement traiter par le borohydrure de sodium pour obtenir le composé de formule **(Ie)** :

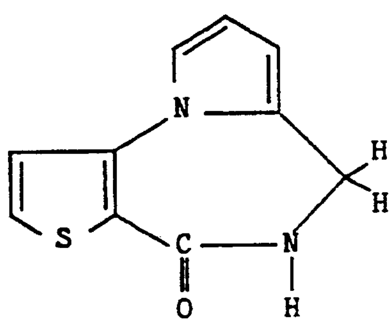

**(Ie)**

que l'on peut éventuellement faire réagir :
a) soit avec un anhydride de formule générale **(XI)**

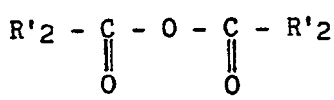

**(XI)**

dans laquelle $R'_2$ représente un radical méthyle ou éthyle de manière à obtenir les composés de formule générale **(II)** :

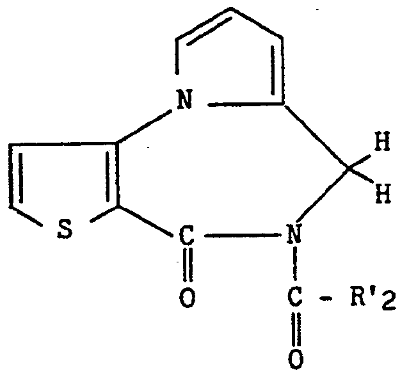

(Il)

dans laquelle $R'_2$ a la signification précedemment définie ;
b) soit avec un isocyanate de formule générale **(XII)** :

$$R_9 - N = C = O \qquad \textbf{(XII)}$$

dans laquelle $R_9$ a la même signification que dans les composés de formule générale **(I)** de manière à obtenir les composés de formule générale **(Im)** :

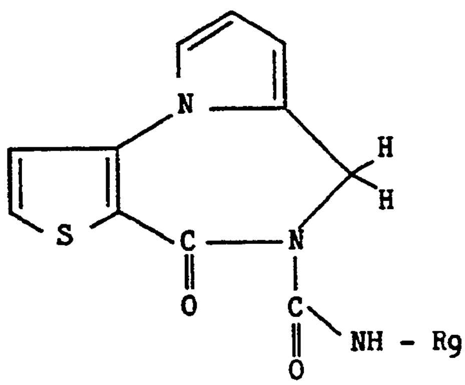

(Im)

dans laquelle $R_9$ à la même signification que dans les composés de formule générale **(I)**,
3) soit en milieu alcalin par une méthyl cétone, de formule générale **(VI)** précedemment définie pour donner les composés de formule générale **(If)** :

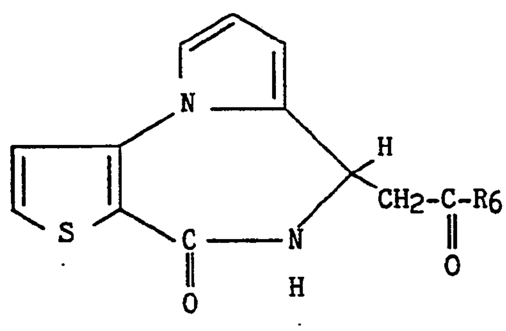

(If)

dans laquelle $R_6$ a la même signification que dans les composés de formule générale **(I)**,
4) soit, en présence de triéthylamine, par un composé de formule générale **(VII)** :

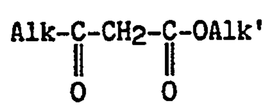

(VII)

dans laquelle Alk et Alk', identiques ou différents, représentent chacun un radical alkyle ayant de 1 à 3

atomes de carbone, pour donner les composés de formule générale **(Ih)** :

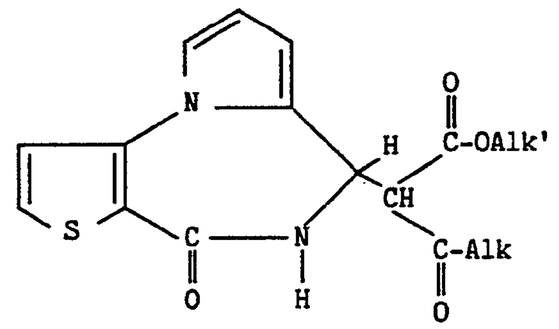

(Ih)

dans laquelle Alk et Alk' ont les significations précédemment définies ;

5) soit, en présence de triéthylamine, par un composé de formule générale **(VIII)** :

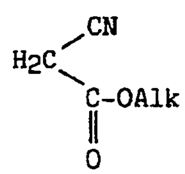

(VIII)

dans laquelle Alk a la signification précédemment définie, pour donner les composés de formule générale **(Ii)** :

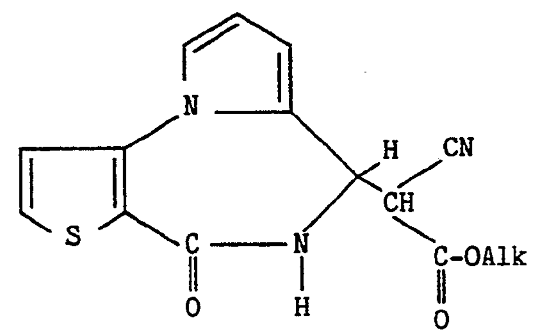

(Ii)

dans laquelle Alk a la signification précédemment définie ;

6) soit par $LiAlH_4$ pour donner le composé de formule **(Ij)** :

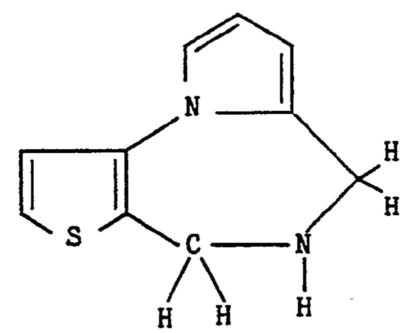

(Ij)

lequel traité

a) soit par une quantité stoechiométrique d'acide minéral ou organique, donne le sel d'addition acide correspondant ;

b) soit par un iodure d'alkyle de formule générale **(IX)** :

$R''_2 I$ **(IX)**

dans laquelle $R''_2$ représente un radical alkyle ayant de 1 à 5 atomes de carbone en chaine droite ou ramifiée donne selon le rapport stoechiométrique **(IX)/(Ij)** :
soit le composé de formule générale **($Ik_1$)** :

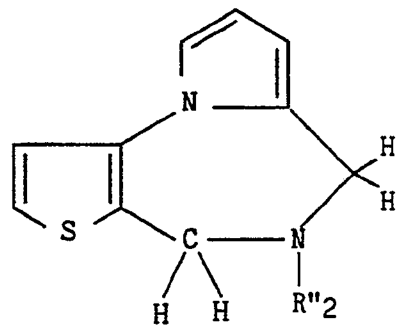

**(Ik1)**

dans laquelle $R''_2$ a la signification précédemment définie,
soit l'iodure de dialkylammonium de formule générale **(In)**

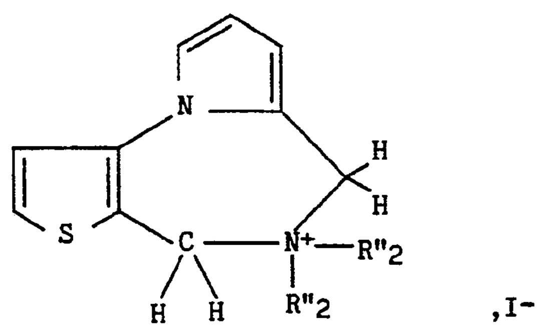

**(In)**

dans laquelle $R''_2$ a la signification précédemment définie,
c) soit par un composé halogené de formule générale **(X)** :

Hal-$R''{}'_2$ **(X)**

dans laquelle $R''{}'_2$ represente un radical arylsulfonyle dans lequel le groupe aryle est éventuellement substitué par un radical alkyle ayant de 1 à 5 atomes de carbone en chaine droite ou ramifiée, donne les composés de formule générale **($Ik_2$)**:

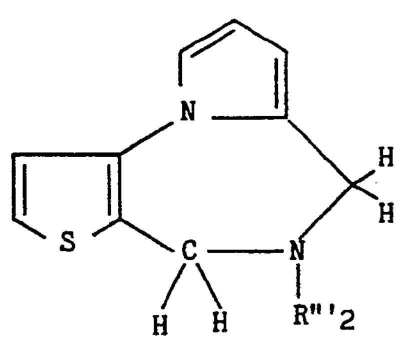

**(Ik2)**

dans laquelle $R''{}'_2$ a la signification précédemment définie,
– Les composés de formule générale **(Ia)** à **(Im)** font partie de l'invention et sont des cas particuliers des composés de formule générale **(I)**.

**22)** Compositions pharmaceutiques contenant comme principe actif un dérivé selon les revendications 1) à 20) seul ou en combinaison avec un ou plusieurs excipients ou véhicules inertes non toxiques pharmaceutiquement acceptables.

**23)** Compositions pharmaceutiques selon la revendication 22) présentées sous une forme convenant

notamment pour le traitement des accidents vasculaires cérébraux, du vieillissement cérébral normal ou pathologique, des syndromes ischémiques, des troubles liés à l'hypoxémie, de l'hypercholestérolémie, de l'hyperlipidémie, de l'hypertriglycéridémie, de l'hyperglycémie, de l'hypertension et des maladies qui leurs sont liées.

**Revendications pour l'Etat contractant suivant : ES**

**1)** Procédé de préparation de nouveaux dérivés de 4H-pyrrolo[1,2-a] thiéno[2,3-f] diazépine [1,4] de formule générale **(I)**:

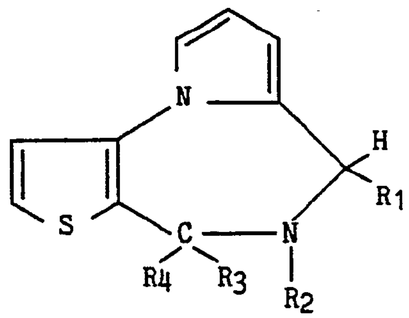

(I)

dans laquelle :

- $R_1$ représente :
  - un atome d'hydrogène,
  - un radical hydroxy,
  - un radical de formule générale :

$$-OR_5 \text{ ou } -SR_5$$

  dans laquelle $R_5$ représente :
  . un radical alkyle contenant de 1 à 5 atomes de carbone, en chaine droite ou ramifiée, éventuellement substitué par un radical phényle ou par un groupe de formule -COOB dans laquelle B représente un atome d'hydrogène ou un radical alkyle contenant de 1 à 5 atomes de carbone, ou
  . un radical aryle tel que par exemple un radical phényle,
  - un radical de formule générale :

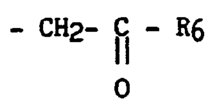 ou 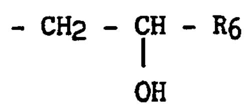

  dans laquelle $R_6$ représente :
  . un radical alkyle linéaire ou ramifié de 1 à 5 atomes de carbone,
  . un radical aryle (éventuellement substitué par un ou plusieurs atomes d'halogènes, groupements alcoxy de 1 à 3 atomes de carbone ou groupements trifluoromethyle),
  . un radical aralkyle de 7 à 9 atomes de carbones (éventuellement substitué sur le noyau aromatique par un ou plusieurs atomes d'halogènes, groupements alcoxy de 1 à 3 atomes de carbone ou groupements trifluorométhyle ),
  - un radical de formule :

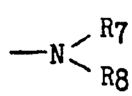

  dans laquelle :
  . $R_7$ et $R_8$, identiques ou différents, représentent chacun un atome d'hydrogène, un radical alkyle contenant de 1 à 5 atomes de carbone en chaine droite ou ramifiée, un radical cycloalkyle contenant de 3 à 5 atomes de carbone, un radical aryle (éventuellement substitué par un ou plusieurs atomes d'halogènes, groupements alcoxy de 1 à 3 atomes de carbone ou groupements trifluorométhyle) ou un radical aralkyle de 7 à 9 atomes de carbone (éventuellement substitué sur le noyau atomatique par un ou plusieurs atomes d'halogènes, groupements alcoxy de 1 à 3 atomes de carbone ou groupements trifluo-

rométhyle) ou,

. $R_7$ et $R_8$ forment ensemble avec l'atome d'azote auquel ils sont liés un radical hétérocyclique penta ou hexagonal renfermant éventuellement un autre hétéroatome tel par exemple un deuxième atome d'azote ou un atome d'oxygène, et éventuellement substitue par un radical alkyle linéaire ou ramifié de 1 à 5 atomes de carbone, par un radical alcoxycarbonyle de 2 à 6 atomes de carbone ou par un radical aryle, aralkyle de 7 à 9 atomes de carbone, hétéroaryle, diaralkyle comme par exemple le radical benzhydryle (éventuellement substitués sur le ou les noyaux aromatiques par un ou plusieurs substituants identiques ou différents choisis parmi halogène, hydroxy, alkyle ou alcoxy de 1 à 4 atomes de carbone, nitro ou trifluorométhyle),

ou

– un radical alkyle renfermant de 1 à 5 atomes de carbone éventuellement substitué par un ou plusieurs radicaux choisis parmis le groupe formé des radicaux hydroxy, oxo, cyano, alkoxycarbonyle renfermant de 2 à 4 atomes de carbone ;

- $R_2$ représente :

– un atome d'hydrogène,

– un radical alkyle ayant de 1 à 5 atomes de carbone en chaine droite ou ramifiée,

– un radical arylsulfonyle dans lequel le groupe aryle est éventuellement substitué par un radical alkyle ayant de 1 à 5 atomes de carbone en chaine droite ou ramifiée ;

– un radical alkylcarbonyle de 2 à 3 atomes de carbone

– un radical de formule générale :

$$-\underset{\substack{||\\ O}}{C}NHR_9$$

dans laquelle

. $R_9$ représente un radical alkyle linéaire ou ramifié, de 1 à 6 atomes de carbone, un radical aryle ou aralkyle de 7 à 9 atomes de carbone (éventuellement substitués sur le noyau aromatique par un ou plusieurs atomes d'halogène, radicaux alcoxy de 1 à 3 atomes de carbone ou radicaux trifluorométhyle),

- $R_3$ et $R_4$ représentent :

– chacun simultanément un atome d'hydrogène ou,

– ensemble un atome d'oxygène,

ainsi que de leurs isomères, diastéréoisomères, énantiomères et de leur sel d'addition à un acide minéral ou organique, pharmaceutiquement acceptable,

caractérisé en ce que :

**A** - soit l'on traite l'aminocarbonyl-2 (formyl-2 pyrrolyl-1)-3 thiophène de formule **(II)** :

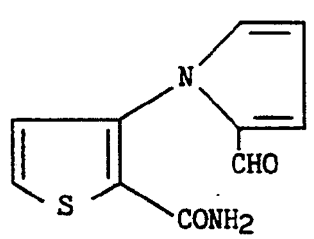

(II)

1) soit avec un mélange eau et triéthylamine, pour donner le composé de formule **(Ia)** :

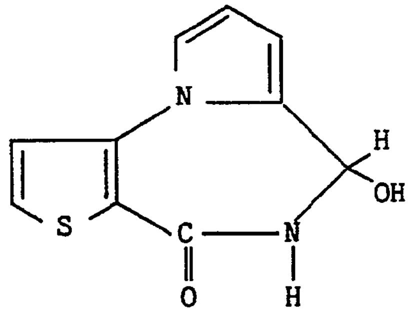

(Ia)

2) soit avec un alcool ou un thiol de formules générales respectives **(III)** et **(IV)** :

$R_5OH$ **(III)**, $R_5SH$ **(IV)**

dans lesquelles $R_5$ a la même signification que dans les composés de formule générale **(I)**, pour donner respectivement les composés de formules générales **(Ib)** et **(Ic)** :

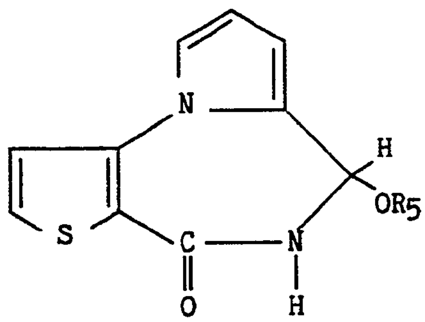

(Ib)

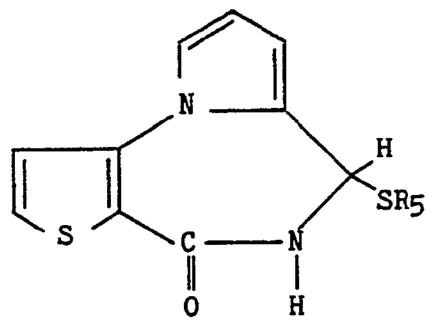

(Ic)

dans lesquelles $R_5$ a la signification précédemment définie ;

3) soit avec une amine secondaire de formule générale **(V)** :

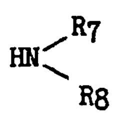

(V)

dans laquelle $R_7$ et $R_8$ ont les mêmes significations que dans les composés de formule générale **(I)**, pour donner les composés de formule générale **(Id)** :

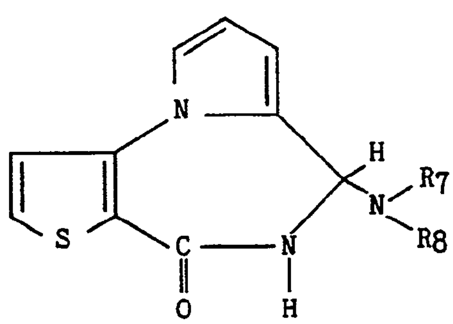

(Id)

dans laquelle $R_7$ et $R_8$ ont les mêmes significations que dans les composés de formule générale **(I)** ;

4) soit en milieu alacalin avec une méthylcétone de formule générale **(VI)** :

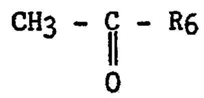

(VI)

dans laquelle $R_6$ a la même signification que dans les composés de formule générale **(I)** de manière à obtenir les composés de formule générale **(If)** :

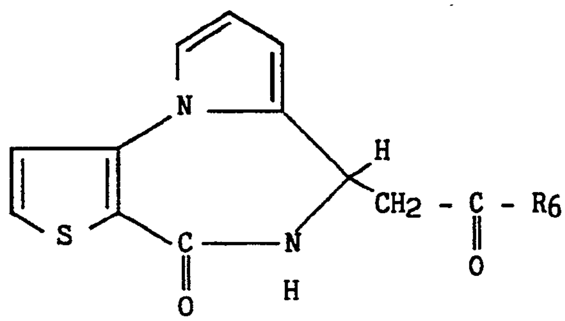

(If)

dans laquelle $R_6$ a la même signification que dans les composés de formule générale **(I)**, que l'on peut éventuellement traiter par un borohydrure de sodium pour obtenir les composés de formule générale **(Ig)** :

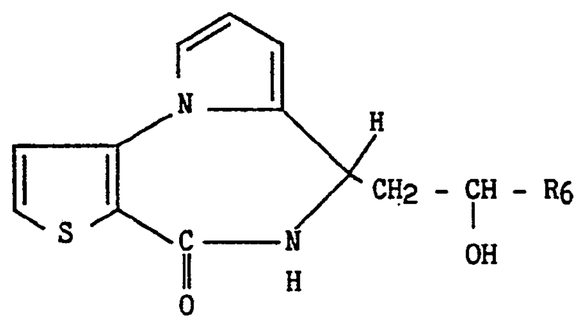

(Ig)

dans laquelle $R_6$ a la même signification que dans les composés de formule générale **(I)** ;

**B** - soit l'on traite le composé de formule générale **(Ia)** précédemment obtenu :

1) soit par un alcool de formule générale **(III)** précédemment définie pour donner les composés de formule générale **(Ib)** :

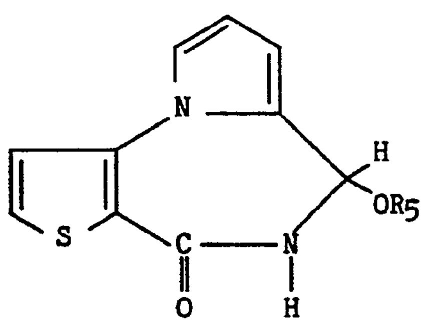

(Ib)

dans laquelle $R_5$ a la même définition que dans les composés de formule générale **(I)** ;

2) soit par une amine de formule générale **(V)** précédemment définie pour donner les composés de formule générale **(Id)** :

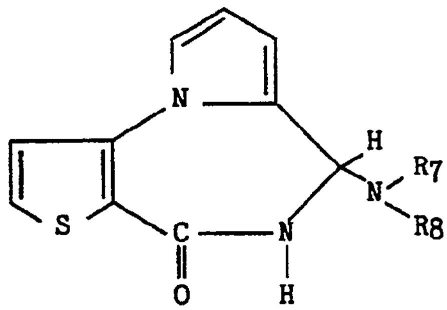

**(Id)**

dans laquelle $R_7$ et $R_8$ ont les mêmes significations que dans les composés de formule générale **(I)**, que l'on peut éventuellement traiter par le borohydrure de sodium pour obtenir le composé de formule **(Ie)** :

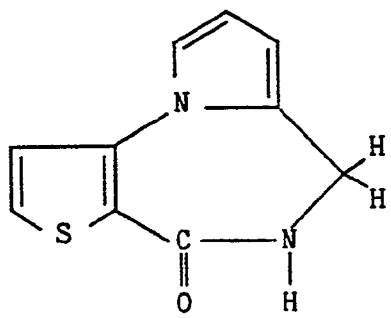

**(Ie)**

que l'on peut éventuellement faire réagir :

a) soit avec un anhydride de formule générale **(XI)**

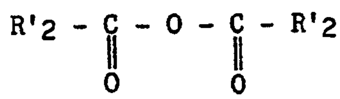

**(XI)**

dans laquelle $R'_2$ représente un radical méthyle ou éthyle de manière à obtenir les composés de formule générale **(Il)** :

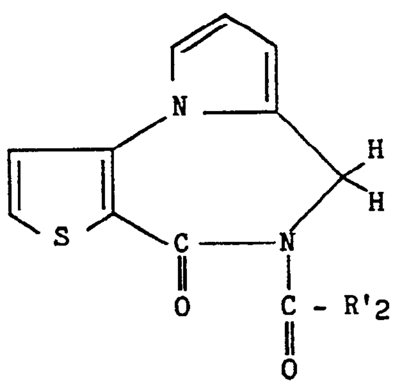

**(Il)**

dans laquelle $R'_2$ a la signification précédemment définie ;

b) soit avec un isocyanate de formule générale **(XII)** :

$$R_9 - N = C = O \quad (XII)$$

dans laquelle $R_9$ a la même signification que dans les composés de formule générale **(I)** de manière à obtenir les composés de formule générale **(Im)** :

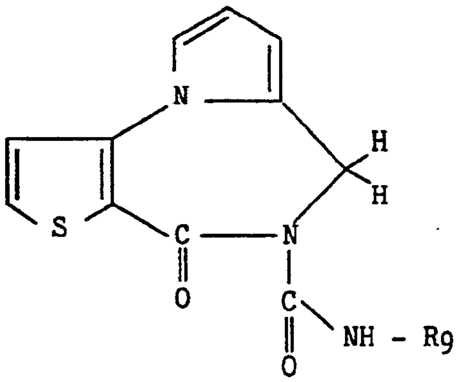

**(Im)**

dans laquelle $R_9$ à la même signification que dans les composés de formule générale **(I)**,

3) soit en milieu alcalin par une méthyl cétone, de formule générale **(VI)** précédemment définie pour donner les composés de formule générale **(If)** :

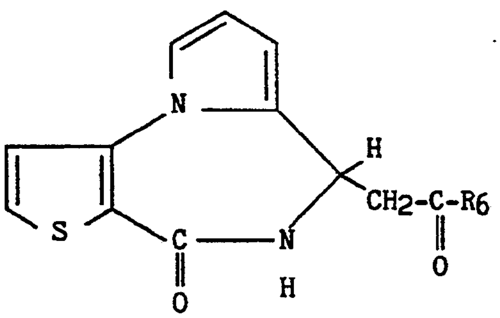

**(If)**

dans laquelle $R_6$ a la même signification que dans les composés de formule générale **(I)**,

4) soit, en présence de triéthylamine, par un composé de formule générale **(VII)** :

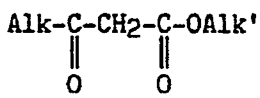

**(VII)**

dans laquelle Alk et Alk', identiques ou différents, représentent chacun un radical alkyle ayant de 1 à 3 atomes de carbone, pour donner les composés de formule générale **(Ih)** :

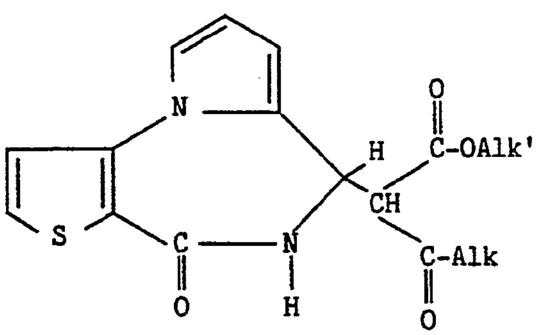

(Ih)

dans laquelle Alk et Alk' ont les significations précédemment définies ;

5) soit, en présence de triéthylamine, par un composé de formule générale **(VIII)** :

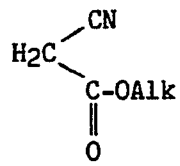

(VIII)

dans laquelle Alk a la signification précédemment définie, pour donner les composés de formule générale **(Ii)** :

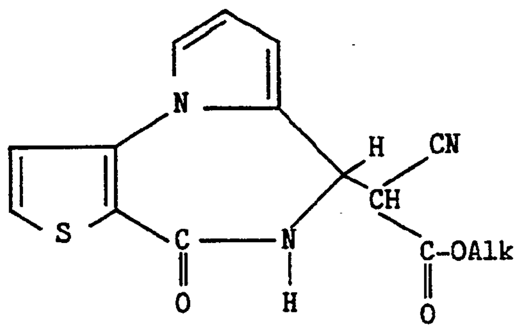

(Ii)

dans laquelle Alk a la signification précédemment définie ;
6) soit par $LiAlH_4$ pour donner le composé de formule **(Ij)** :

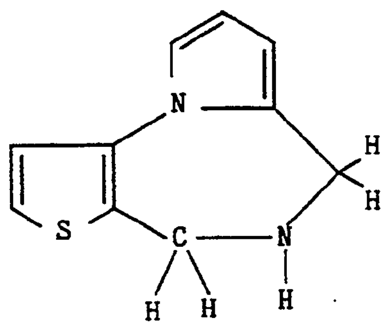

(Ij)

lequel traité

a) soit par une quantité stoechiométrique d'acide minéral ou organique, donne le sel d'addition acide correspondant ;

b) soit par un iodure d'alkyle de formule générale **(IX)** :

$R''_2 I$ **(IX)**

dans laquelle $R''_2$ représente un radical alkyle ayant de 1 à 5 atomes de carbone en chaine droite ou ramifiée donne selon le rapport stoechiométrique **(IX)/(Ij)** :

soit le composé de formule générale **($Ik_1$)** :

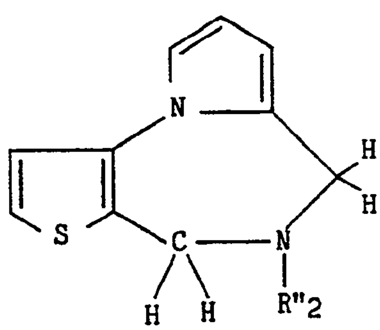

($Ik_1$)

dans laquelle $R''_2$ a la signification précédemment définie,
soit l'iodure de dialkylammonium de formule générale **(In)**

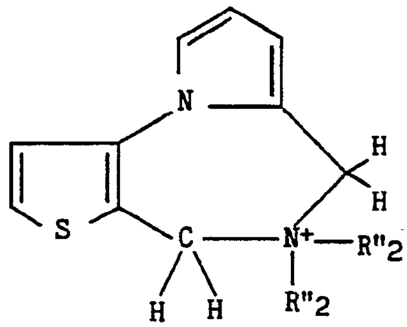

(In)

,I-

dans laquelle R"$_2$ a la signification précédemment définie,

c) soit par un composé halogéné de formule générale **(X)** :

Hal-R" '$_2$ **(X)**

dans laquelle R" '$_2$ représente un radical arylsulfonyle dans lequel le groupe aryle est éventuellement substitué par un radical alkyle ayant de 1 à 5 atomes de carbone en chaine droite ou ramifiée, donne les composés de formule générale **(Ik$_2$)**:

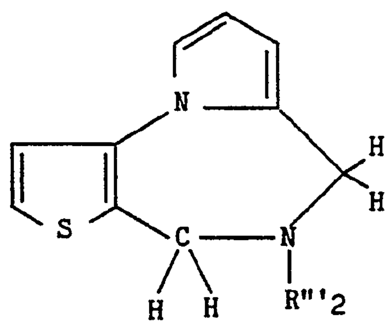

(Ik2)

dans laquelle R" '$_2$ a la signification précédemment définie.

**2)** Procédé de préparation selon la revendication 1) du composé qui est l'hydroxy-6 dihydro-5,6 oxo-4 4H-pyrrolo[1,2-a] thieno[2,3-f] diazepine[1,4], ainsi que de ses isomères isolés ou sous forme de mélange.

**3)** Procédé de préparation selon la revendication 1) du composé qui est la méthoxy-6 dihydro-5,6 oxo-4 4H-pyrrolo[1,2-a] thiéno[2,3-f] diazépine[1,4] ainsi que de ses isomères isolés ou sous forme de mélange.

**4)** Procédé de préparation selon la revendication 1) du composé qui est la méthoxycarbonylméthylmercapto-6 dihydro-5,6 oxo-4 4H-pyrrolo [1,2-a] thiéno[2,3-f] diazépine[1,4] ainsi que de ses isomères isolés ou sous forme de mélange.

**5)** Procédé de préparation selon la revendication 1) du composé qui est la méthylamino-6 dihydro-5,6 oxo-4 4H-pyrrolo[1,2-a] thiéno[2,3-f] diazépine[1,4] ainsi que de ses isomères isolés ou sous forme de mélange et de leurs sels d'addition à un acide pharmaceutiquement acceptable.

**6)** Procédé de préparation selon la revendication 1) du composé qui est la dihydro-5,6 oxo-4 4H-pyrrolo[1,2-a] thieno[2,3-f] diazépine[1,4].

**7)** Procédé de préparation selon la revendication 1) du composé qui est l'acétonyl-6 dihydro-5,6 oxo-4 4H-pyrrolo[1,2-a] thiéno[2,3-f] diazépine[1,4], ainsi que de ses isomères isolés ou sous forme de mélange.

**8)** Procédé de préparation selon la revendication 1) du composé qui est l'(hydroxy-2 propyl)-6 dihydro-5,6 oxo-4 4H-pyrrolo[1,2-a] thiéno[2,3-f] diazépine[1,4], ainsi que de ses isomères isolés ou sous forme de mélange.

**9)** Procédé de préparation selon la revendication 1) du composé qui est l'(ethoxycarbonyl-cyanométhyl)-6 dihydro-5,6 oxo-4 4H-pyrrolo[1,2-a] thiéno[2,3-f] diazépine[1,4], ainsi que de ses isomères isoles ou sous forme de mélange..

**10)** Procédé de préparation selon la revendication 1) du composé qui est la (methoxycarbonyl-cyanométhyl)-6 dihydro-5,6 oxo-4 4H-pyrrolo[1,2-a] thiéno[2,3-f] diazepine[1,4], ainsi que de ses isomères isolés ou sous forme de mélange.

**11)** Procédé de préparation selon la revendication 1) du composé qui est l'(éthoxycarbonyl-1 acétonyl)-6 dihydro-5,6 oxo-4 4H-pyrrolo [1,2-a] thiéno[2,3-f] diazépine[1,4], ainsi que de ses isomères isolés ou sous forme de mélange.

**12)** Procédé de préparation selon la revendication 1) du composé qui est la dihydro-5,6 4H-pyrrolo[1,2-a]

thieno[2,3-f] diazépine[1,4] ainsi que de ses sels d'addition à un acide pharmaceutiquement acceptable.

**13)** Procédé de préparation selon la revendication 1) du composé qui est la p.tolylsulfonyl-5 dihydro-5,6 4H-pyrrolo[1,2-a] thieno[2,3-f] diazépine[1,4].

**14)** Procédé de préparation selon la revendication 1) du composé qui est l'acetyl-5 dihydro-5,6 oxo-4 4H-pyrrolo[1,2-a] thieno[2,3-f] diazépine[1,4].

**15)** Procédé de préparation selon la revendication 1) du composé qui est la (N-phenyl carbamoyl)-5 dihydro-5,6 oxo-4 4H-pyrrolo[1,2-a] thiéno[2,3-f] diazépine[1,4].

**16)** Procédé de préparation selon la revendication 1) du composé qui est la phénacyl-6 dihydro-5,6 oxo-4 4H-pyrrolo[1,2-a] thiéno[2,3-f] diazépine[1,4], ainsi que de ses isomères isolés ou sous forme de mélange.

**17)** Procédé de préparation selon la revendication 1) du composé qui est la morpholino-6 dihydro-5,6 oxo-4 4H-pyrrolo[1,2-a] thiéno[2,3-f] diazépine[1,4], ainsi que de ses isomères isolés ou sous forme de mélange et de leurs sels d'addition à un acide pharmaceutiquement acceptable.

**18)** Procédé de préparation selon la revendication 1) du composé qui est la pyrrolidino-6 dihydro-5,6 oxo-4 4H-pyrrolo[1,2-a] thieno[2,3-f] diazépine[1,4], ainsi que de ses isomères isolés ou sous forme de mélange et de leurs sels d'addition à un acide pharmaceutiquement acceptable.

**19)** Procédé de préparation des compositions pharmaceutiques contenant comme principe actif un dérivé préparé selon les revendications 1) à 18) seul ou en combinaison avec un ou plusieurs excipients ou véhicules inertes non toxiques pharmaceutiquement acceptables.

**20)** Procédé de préparation des compositions pharmaceutiques selon la revendication 19) présentées sous une forme convenant notamment pour le traitement des accidents vasculaires cérébraux, du vieillissement cérébral normal ou pathologique, des syndromes ischémiques, des troubles liés à l'hypoxémie, de l'hypercholestérolémie, de l'hyperlipidémie, de l'hypertriglycéridémie, de l'hyperglycémie, de l'hypertension et des maladies qui leurs sont liées.

**Revendications pour l'Etat contractant suivant : GR**

**1)** Procédé de préparation de nouveaux dérivés de 4H-pyrrolo[1,2-a] thiéno[2,3-f] diazépine [1,4] de formule générale **(I):**

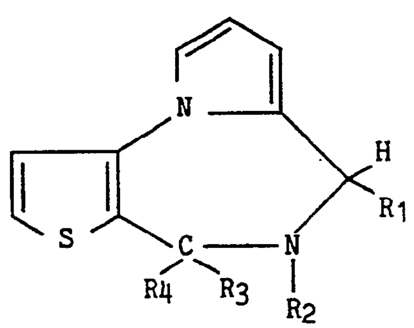

(I)

dans laquelle :

- $R_1$ représente :
  - un atome d'hydrogène,
  - un radical hydroxy,
  - un radical de formule générale :

$$-OR_5 \text{ ou } -SR_5$$

dans laquelle $R_5$ représente :

. un radical alkyle contenant de 1 à 5 atomes de carbone, en chaine droite ou ramifiée, éventuellement substitué par un radical phényle ou par un groupe de formule -COOB dans laquelle B représente un atome d'hydrogène ou un radical alkyle contenant de 1 à 5 atomes de carbone, ou

. un radical aryle tel que par exemple un radical phényle,

  - un radical de formule générale :

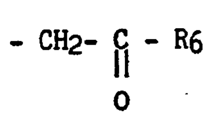

ou

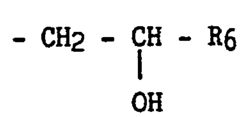

dans laquelle $R_6$ représente :

. un radical alkyle linéaire ou ramifie de 1 à 5 atomes de carbone,

. un radical aryle (éventuellement substitué par un ou plusieurs atomes d'halogènes, groupements alcoxy de 1 à 3 atomes de carbone ou groupements trifluoromethyle ),

. un radical aralkyle de 7 à 9 atomes de carbones (éventuellement substitué sur le noyau aromatique par un ou plusieurs atomes d'halogènes, groupements alcoxy de 1 à 3 atomes de carbone ou groupements trifluorométhyle),

– un radical de formule :

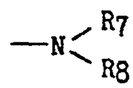

dans laquelle :

. $R_7$ et $R_8$, identiques ou différents, représentent chacun un atome d'hydrogène, un radical alkyle contenant de 1 à 5 atomes de carbone en chaine droite ou ramifiée, un radical cycloalkyle contenant de 3 à 5 atomes de carbone, un radical aryle (éventuellement substitué par un ou plusieurs atomes d'halogènes, groupements alcoxy de 1 à 3 atomes de carbone ou groupements trifluorométhyle) ou un radical aralkyle de 7 à 9 atomes de carbone (éventuellement substitué sur le noyau atomatique par un ou plusieurs atomes d'halogènes, groupements alcoxy de 1 à 3 atomes de carbone ou groupements trifluorométhyle) ou,

. $R_7$ et $R_8$ forment ensemble avec l'atome d'azote auquel ils sont liés un radical hétérocyclique penta ou hexagonal renfermant éventuellement un autre hétéroatome tel par exemple un deuxième atome d'azote ou un atome d'oxygène, et éventuellement substitué par un radical alkyle linéaire ou ramifié de 1 à 5 atomes de carbone, par un radical alcoxycarbonyle de 2 à 6 atomes de carbone ou par un radical aryle, aralkyle de 7 à 9 atomes de carbone, hétéroaryle, diaralkyle comme par exemple le radical benzhydryle (éventuellement substitués sur le ou les noyaux aromatiques par un ou plusieurs substituants identiques ou différents choisis parmi halogène, hydroxy, alkyle ou alcoxy de 1 à 4 atomes de carbone, nitro ou trifluorométhyle),

ou

– un radical alkyle renfermant de 1 à 5 atomes de carbone éventuellement substitué par un ou plusieurs radicaux choisis parmis le groupe formé des radicaux hydroxy, oxo, cyano, alkoxycarbonyle renfermant de 2 à 4 atomes de carbone ;

- $R_2$ représente :

– un atome d'hydrogène,

– un radical alkyle ayant de 1 à 5 atomes de carbone en chaine droite ou ramifiée,

– un radical arylsulfonyle dans lequel le groupe aryle est éventuellement substitué par un radical alkyle ayant de 1 à 5 atomes de carbone en chaine droite ou ramifiée ;

– un radical alkylcarbonyle de 2 à 3 atomes de carbone

– un radical de formule générale :

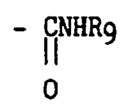

dans laquelle

. $R_9$ représente un radical alkyle linéaire ou ramifié, de 1 à 6 atomes de carbone, un radical aryle ou aralkyle de 7 à 9 atomes de carbone (éventuellement substitués sur le noyau aromatique par un ou plusieurs atomes d'halogène, radicaux alcoxy de 1 à 3 atomes de carbone ou radicaux trifluoromethyle),

- $R_3$ et $R_4$ représentent :

– chacun simultanément un atome d'hydrogène ou,

– ensemble un atome d'oxygène,

ainsi que de leurs isomères, diastéréoisomères, énantiomères et de leur sel d'addition à un acide minéral ou organique, pharmaceutiquement acceptable,

caractérisé en ce que :

**A** - soit l'on traite l'aminocarbonyl-2 (formyl-2 pyrrolyl-1)-3 thiophène de formule **(II)** :

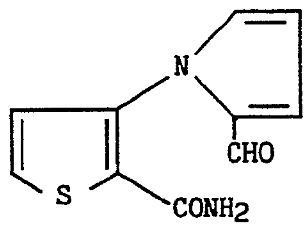

**(II)**

1) soit avec un mélange eau et triéthylamine, pour donner le composé de formule **(Ia)** :

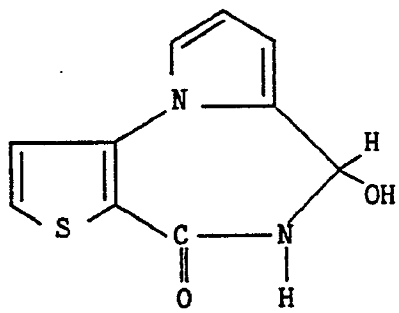

**(Ia)**

2) soit avec un alcool ou un thiol de formules générales respectives **(III)** et **(IV)** :

$R_5OH$ **(III)**, $R_5SH$ **(IV)**

dans lesquelles $R_5$ a la même signification que dans les composés de formule générale **(I)**, pour donner respectivement les composés de formules générales **(Ib)** et **(Ic)** :

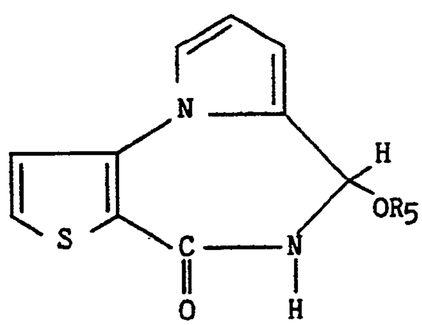

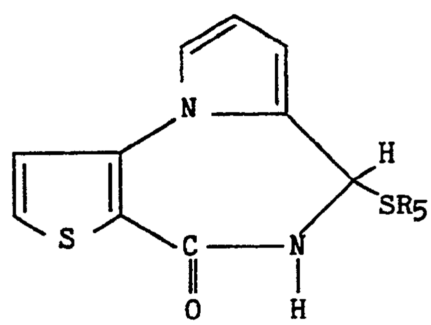

**(Ib)** **(Ic)**

dans lesquelles $R_5$ a la signification précédemment définie ;

3) soit avec une amine secondaire de formule générale **(V)** :

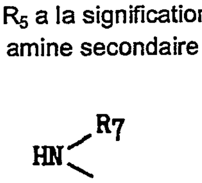

**(V)**

dans laquelle $R_7$ et $R_8$ ont les mêmes significations que dans les composés de formule générale **(I)**, pour donner les composés de formule générale **(Id)** :

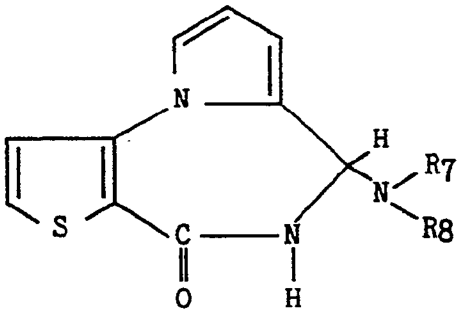

(Id)

dans laquelle $R_7$ et $R_8$ ont les mêmes significations que dans les composés de formule générale **(I)** ;

4) soit en milieu alacalin avec une méthylcétone de formule générale **(VI)** :

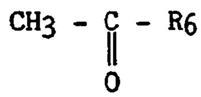

(VI)

dans laquelle $R_6$ a la même signification que dans les composés de formule générale **(I)** de manière à obtenir les composés de formule générale **(If)** :

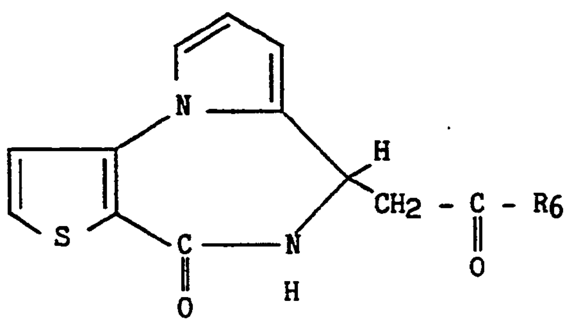

(If)

dans laquelle $R_6$ a la même signification que dans les composés de formule générale **(I)**, que l'on peut éventuellement traiter par un borohydrure de sodium pour obtenir les composés de formule générale **(Ig)** :

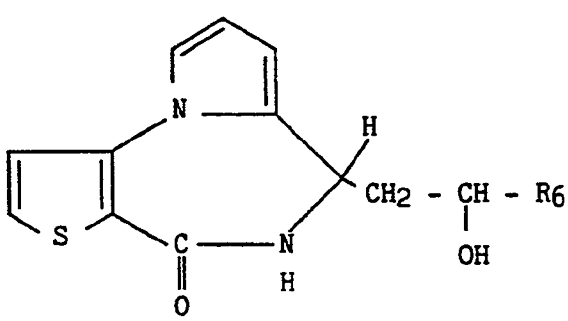

(Ig)

dans laquelle $R_6$ a la même signification que dans les composés de formule générale **(I)** ;

**B** - soit l'on traite le composé de formule générale **(Ia)** précédemment obtenu :

1) soit par un alcool de formule générale **(III)** précédemment définie pour donner les composes de formule générale **(Ib)** :

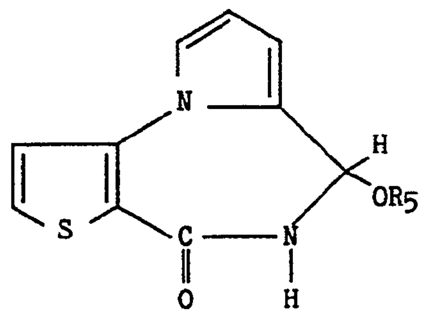

(Ib)

dans laquelle $R_5$ a la même définition que dans les composés de formule générale **(I)** ;

2) soit par une amine de formule générale **(V)** précédemment définie pour donner les composés de formule générale **(Id)** :

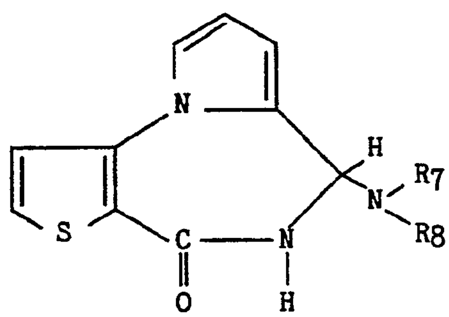

(Id)

dans laquelle $R_7$ et $R_8$ ont les mêmes significations que dans les composés de formule générale **(I)**, que l'on peut éventuellement traiter par le borohydrure de sodium pour obtenir le composé de formule **(Ie)** :

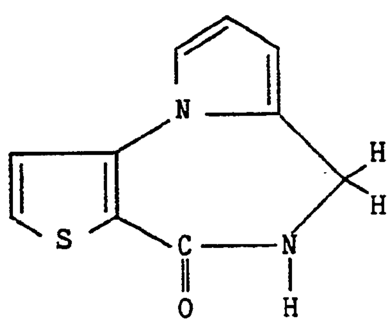

(Ie)

que l'on peut éventuellement faire réagir :

a) soit avec un anhydride de formule générale **(XI)**

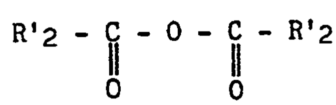

(XI)

dans laquelle $R'_2$ représente un radical méthyle ou éthyle de manière à obtenir les composés de formule générale **(II)** :

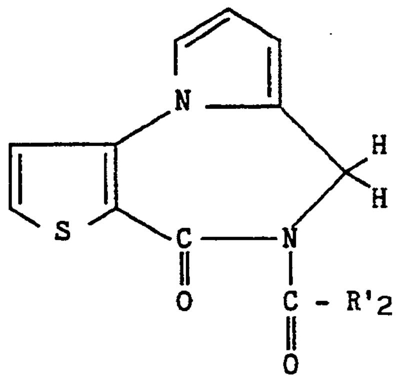

(I1)

dans laquelle $R'_2$ a la signification précédemment définie ;
b) soit avec un isocyanate de formule générale **(XII)** :

$R_9 - N = C = O$ **(XII)**

dans laquelle $R_9$ a la même signification que dans les composés de formule générale **(I)** de manière à obtenir les composés de formule générale **(Im)** :

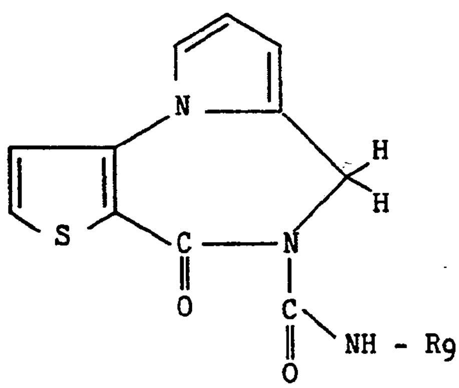

(Im)

dans laquelle $R_9$ à la même signification que dans les composés de formule générale **(I)**,
3) soit en milieu alcalin par une méthyl cétone, de formule générale **(VI)** précédemment définie pour donner les composés de formule générale **(If)** :

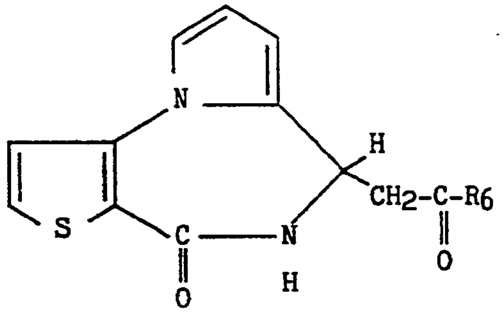

(If)

dans laquelle $R_6$ a la même signification que dans les composés de formule générale **(I)**,
4) soit, en présence de triéthylamine, par un compose de formule générale **(VII)** :

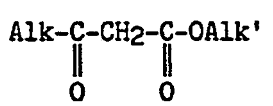

(VII)

dans laquelle Alk et Alk', identiques ou différents, représentent chacun un radical alkyle ayant de 1 à 3

atomes de carbone, pour donner les composés de formule générale **(Ih)** :

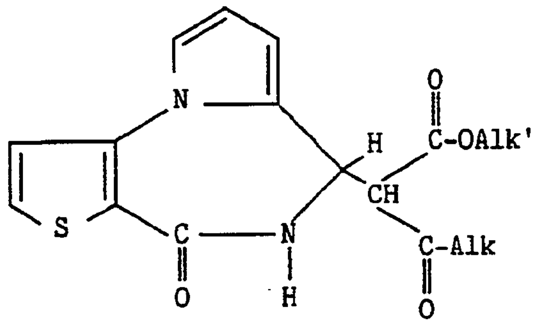

(Ih)

dans laquelle Alk et Alk' ont les significations précédemment définies ;
5) soit, en présence de triéthylamine, par un composé de formule générale **(VIII)** :

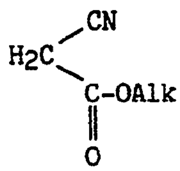

**(VIII)**

dans laquelle Alk a la signification précédemment définie, pour donner les composés de formule générale **(Ii)** :

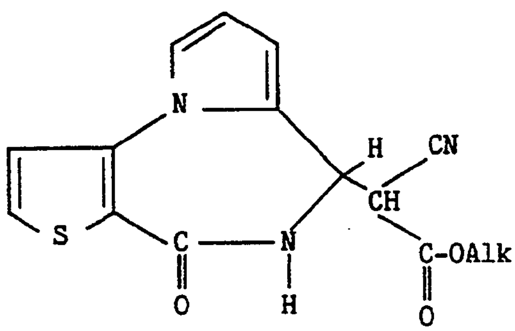

**(Ii)**

dans laquelle Alk a la signification précédemment définie ;
6) soit par $LiAlH_4$ pour donner le composé de formule **(Ij)** :

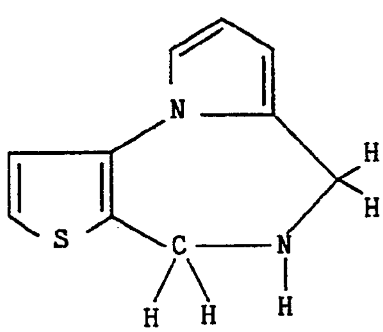

**(Ij)**

lequel traité

a) soit par une quantité stoechiométrique d'acide minéral ou organique, donne le sel d'addition acide correspondant ;

b) soit par un iodure d'alkyle de formule générale **(IX)** :

$R''_2 I$ **(IX)**

dans laquelle R″$_2$ représente un radical alkyle ayant de 1 à 5 atomes de carbone en chaine droite ou ramifiée donne selon le rapport stoechiométrique **(IX)/(Ij)** :
soit le composé de formule générale **(Ik$_1$)** :

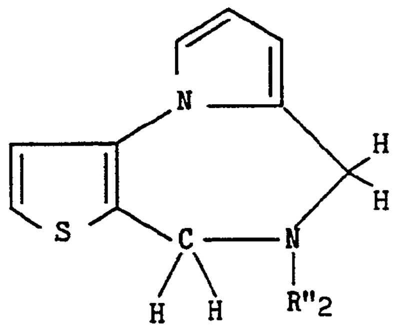

**(Ik1)**

dans laquelle R″$_2$ a la signification précédemment définie,
soit l'iodure de dialkylammonium de formule générale **(In)**

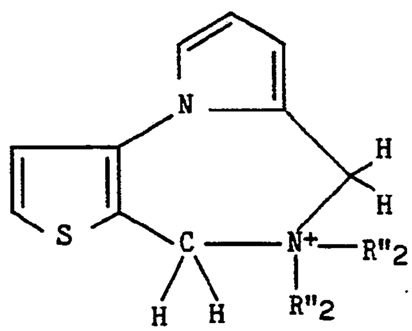

,I-

**(In)**

dans laquelle R″$_2$ a la signification précédemment définie,
c) soit par un composé halogéné de formule générale **(X)** :

Hal-R″ ′$_2$ **(X)**

dans laquelle R″ ′$_2$ représente un radical arylsulfonyle dans lequel le groupe aryle est éventuellement substitué par un radical alkyle ayant de 1 à 5 atomes de carbone en chaine droite ou ramifiée, donne les composés de formule générale **(Ik$_2$)**:

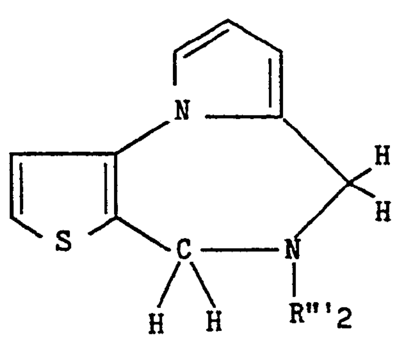

**(Ik2)**

dans laquelle R″ ′$_2$ a la signification précédemment définie.

**2)** Procédé de préparation selon la revendication 1) du composé qui est l'hydroxy-6 dihydro-5,6 oxo-4 4H-pyrrolo[1,2-a] thieno[2,3-f] diazépine[1,4], ainsi que de ses isomères isolés ou sous forme de mélange.

**3)** Procédé de préparation selon la revendication 1) du composé qui est la méthoxy-6 dihydro-5,6 oxo-4 4H-pyrrolo[1,2-a] thiéno[2,3-f] diazepine[1,4] ainsi que de ses isomères isolés ou sous forme de mélange.

**4)** Procédé de préparation selon la revendication 1) du composé qui est la méthoxycarbonylméthylmercapto-6 dihydro-5,6 oxo-4 4H-pyrrolo [1,2-a] thiéno[2,3-f] diazépine[1,4] ainsi que de ses isomères isolés ou sous forme de mélange.

**5)** Procédé de préparation selon la revendication 1) du composé qui est la méthylamino-6 dihydro-5,6 oxo-4 4H-pyrrolo[1,2-a] thiéno[2,3-f] diazépine[1,4] ainsi que de ses isomères isolés ou sous forme de mélange et de leurs sels d'addition à un acide pharmaceutiquement acceptable.

**6)** Procédé de préparation selon la revendication 1) du composé qui est la dihydro-5,6 oxo-4 4H-pyrrolo[1,2-a] thieno[2,3-f] diazepine[1,4].

**7)** Procédé de préparation selon la revendication 1) du composé qui est l'acétonyl-6 dihydro-5,6 oxo-4 4H-pyrrolo[1,2-a] thiéno[2,3-f] diazépine[1,4], ainsi que de ses isomères isolés ou sous forme de mélange.

**8)** Procédé de préparation selon la revendication 1) du composé qui est l'(hydroxy-2 propyl)-6 dihydro-5,6 oxo-4 4H-pyrrolo[1,2-a] thiéno[2,3-f] diazépine[1,4], ainsi que de ses isomères isolés ou sous forme de mélange.

**9)** Procédé de préparation selon la revendication 1) du composé qui est l'(éthoxycarbonyl-cyanométhyl)-6 dihydro-5,6 oxo-4 4H-pyrrolo[1,2-a] thiéno[2,3-f] diazépine[1,4], ainsi que de ses isomères isolés ou sous forme de mélange..

**10)** Procédé de préparation selon la revendication 1) du composé qui est la (méthoxycarbonyl-cyanométhyl)-6 dihydro-5,6 oxo-4 4H-pyrrolo[1,2-a] thiéno[2,3-f] diazépine[1,4], ainsi que de ses isomères isolés ou sous forme de mélange.

**11)** Procédé de préparation selon la revendication 1) du composé qui est l'(ethoxycarbonyl-1 acétonyl)-6 dihydro-5,6 oxo-4 4H-pyrrolo [1,2-a] thiéno[2,3-f] diazépine[1,4], ainsi que de ses isomères isolés ou sous forme de mélange.

**12)** Procédé de préparation selon la revendication 1) du composé qui est la dihydro-5,6 4H-pyrrolo[1,2-a] thiéno[2,3-f] diazépine[1,4] ainsi que de ses sels d'addition à un acide pharmaceutiquement acceptable.

**13)** Procédé de préparation selon la revendication 1) du composé qui est la p.tolylsulfonyl-5 dihydro-5,6 4H-pyrrolo[1,2-a] thiéno[2,3-f] diazépine[1,4].

**14)** Procédé de préparation selon la revendication 1) du composé qui est l'acétyl-5 dihydro-5,6 oxo-4 4H-pyrrolo[1,2-a] thiéno[2,3-f] diazépine[1,4].

**15)** Procédé de préparation selon la revendication 1 ) du composé qui est la (N-phényl- carbamoyl)-5 dihydro-5,6 oxo-4 4H-pyrrolo [1,2-a] thiéno [2,3-f] diazépine[1,4].

**16)** Procédé de préparation selon la revendication 1) du composé qui est la phénacyl-6 dihydro-5,6 oxo-4 4H-pyrrolo[1,2-a] thiéno[2,3-f] diazépine[1,4], ainsi que de ses isomères isolés ou sous forme de mélange.

**17)** Procédé de préparation selon la revendication 1) du composé qui est la morpholino-6 dihydro-5,6 oxo-4 4H-pyrrolo[1,2-a] thiéno[2,3-f] diazepine[1,4], ainsi que de ses isomères isolés ou sous forme de mélange et de leurs sels d'addition à un acide pharmaceutiquement acceptable.

**18)** Procedé de préparation selon la revendication 1) du composé qui est la pyrrolidino-6 dihydro-5,6 oxo-4 4H-pyrrolo[1,2-a] thiéno[2,3-f] diazépine[1,4], ainsi que de ses isomères isolés ou sous forme de mélange et de leurs sels d'addition à un acide pharmaceutiquement acceptable.

**19)** Procédé de préparation des compositions pharmaceutiques contenant comme principe actif un dérive prépare selon les revendications 1) à 18) seul ou en combinaison avec un ou plusieurs excipients ou véhicules inertes non toxiques pharmaceutiquement acceptables.

**20)** Procédé de préparation des compositions pharmaceutiques selon la revendication 19) présentées sous une forme convenant notamment pour le traitement des accidents vasculaires cérébraux, du vieillissement cérébral normal ou pathologique, des syndromes ischémiques, des troubles liés à l'hypoxémie, de l'hypercholestérolémie, de l'hyperlipidémie, de l'hypertriglycéridemie, de l'hyperglycémie, de l'hypertension et des maladies qui leurs sont liées.

Office européen des brevets

# RAPPORT DE RECHERCHE EUROPEENNE

Numero de la demande

EP 91 40 0822

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 027 214 (HOFFMANN LA ROCHE)<br>* Revendications 1,35,36 *<br>--- | 1,22,23 | C 07 D 495/14<br>A 61 K 31/55 //<br>(C 07 D 495/14<br>C 07 D 333:00<br>C 07 D 243:00<br>C 07 D 209:00 ) |
| A | CHEMICAL ABSTRACTS, vol. 101, no. 19, 5 novembre 1984, page 700, résumé no. 171222g, Columbus, Ohio, US; F. CORELLI et al.: "Heterocyclic systems. V. Synthesis and CNS activity of derivatives of 6H-pyrrolo[1,2-a][1,4]benzodiazepinne" & FARMACO, ED. SCI. 1984, 39(8), 707-17<br>* Abrégé *<br>--- | 1 | |
| A | COMPTES RENDUS DE L'ACADEMIE DES SCIENCES, vol. 287, serie C, no. 4, 11 septembre 1978, pages 117-120, Académie des Sciences, Paris, FR; S. RAULT et al.: "Sythèse et étude de la 4H-dihydro-5.6 pyrrolo [1.2-a] thiéno [3.2-f] diazépine-1.4"<br>* Page 117 *<br>----- | 1 | **DOMAINES TECHNIQUES RECHERCHES (Int. Cl.5)**<br>C 07 D 495/00<br>A 61 K 31/00 |

Le présent rapport a été établi pour toutes les revendications

| Lieu de la recherche | Date d'achèvement de la recherche | Examinateur |
|---|---|---|
| LA HAYE | 27-05-1991 | VOYIAZOGLOU D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)